# EUROPÄISCHE PATENTSCHRIFT

(11) **EP 0 498 179 B1**
(45) Veröffentlichungstag und Bekanntmachung des Hinweises auf die Patenterteilung: **25.09.1996**
(21) Anmeldenummer: 92100626.8
(22) Anmeldetag: 16.01.1992
(51) Int. Cl.: C07D 261/08, C07D 285/12, C07D 271/10, C07C 233/60, A01N 53/00

(54) **Cyclopropancarbonsäureamide und -thioamide, Verfahren und Zwischenprodukte zu ihrer Herstellung sowie ihre Verwendung zur Schädlingsbekämpfung**
Cyclopropancarboxylic acid amides and thioamides, process and intermediates for their preparation and their use as pesticides
Amides et thioamides d'acides cyclopropanecarboxyliques procédé et intermédiaires de préparation et leur utilisation comme pesticides

(30) Priorität: 05.02.1991 DE 4103382
(43) Veröffentlichungstag der Anmeldung: 12.08.1992
(73) Patentinhaber: BASF Aktiengesellschaft, 67063 Ludwigshafen (DE)
(72) Erfinder: Nuebling, Christoph, Dr., W-6733 Hassloch (DE); Theobald, Hans, Dr., W-6703 Limburgerhof (DE); Kardorff, Uwe, Dr., W-6800 Mannheim 1 (DE); Krieg, Wolfgang, Dr., W-6721 Weingarten (DE); Kuenast, Christoph, Dr., W-6701 Otterstadt (DE)

(56) Entgegenhaltungen:
- EP-A- 0 285 934
- EP-A- 0 350 688
- EP-A- 0 351 617
- EP-A- 0 353 571

## Beschreibung

Die vorliegende Erfindung betrifft Cyclopropancarbonsäureamide und -thioamide der allgemeinen Formel I, 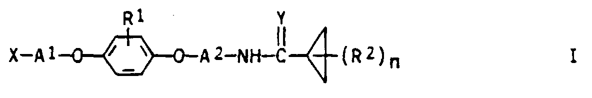 in der der Index und die Substituenten folgende Bedeutung haben:
- n: 0, 1 oder 2, wobei die Reste R² verschieden sein können, wenn n für 2 steht;
- A¹: Methylen, Ethylen oder Propylen, wobei diese Gruppen ein oder zwei C₁-C₃-Alkylgruppen tragen können;
- A²: Ethylen oder Propylen, wobei diese Gruppen ein oder zwei C₁-C₃-Alkylgruppen tragen können;
- R¹: Wasserstoff, Halogen oder C₁-C₆-Alkyl;
- R²: Halogen oder C₁-C₃-Alkyl;
- X: ein über ein C-Atom oder ein N-Atom gebundener 5-gliedriger heteroaromatischer Rest, enthaltend ein bis drei Stickstoffatome oder ein Sauerstoff- oder ein Schwefelatom oder enthaltend ein oder zwei Stickstoffatome und ein Sauerstoff- oder ein Schwefelatom als Heteroatome, oder ein Pyrazolyl-1-oxy- oder Triazolyl-1-oxyrest, wobei diese 5-Ring Heteroaromaten an ihren Kohlenstoffringgliedern ein bis drei der der folgenden Reste tragen können: Nitro, Cyano, Halogen, C₁-C₆-Alkyl, C₁-C₄-Halogenalkyl, C₁-C₆-Alkoxy, C₁-C₄-Halogenalkoxy, C₁-C₄-Alkylthio, C₁-C₄-Halogenaikylthio, C₁-C₄-Alkoxy-C₁-C₄-alkyl, C₃-C₈-Cycloalkyl, C₂-C₈-Alkenyl, C₂-C₈-Alkinyl, Aryl oder Aryl-C₁-C₁₀-alkyl, wobei die aromatischen Reste ihrerseits ein bis fünf Halogenatome und/oder ein bis drei der folgenden Gruppen tragen können: C₁-C₄-Alkyl, C₁-C₄-Halogenalkyl, C₁-C₄-Alkoxy, C₁-C₄-Halogenalkoxy, C₁-C₄-Alkylthio oder C₁-C₄-Halogenalkylthio;
- Y: Sauerstoff oder Schwefel.

Außerdem betrifft die Erfindung Verfahren zur Herstellung dieser Verbindungen, sie enthaltende Schädlingsbekämpfungsmittel sowie Verfahren zur Schädlingsbekämpfung.

Desweiteren betrifft die Erfindung Zwischenprodukte zur Herstellung der Cyclopropancarbonsäureamide und -thioamide I, nämlich Phenole der allgemeinen Formel VIII, 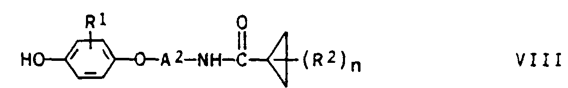 in der der Index n und die Substituenten A², R¹ und R² die vorstehend gegebene Bedeutung haben sowie Amine der allgemeinen Formel X, 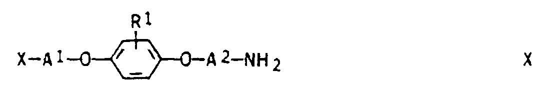 in der die Substituenten A¹, A², R¹ und X die vorstehend gegebene Bedeutung haben.

Aus der Literatur sind Cyclopropancarbonsäureamide mit schädlingsbekämpfender Wirkung bekannt (Cyclopropancarbonsäure-N-phenoxyphenoxyalkylamide: EP-A 258 733, EP-A 350 688 und DE-A 38 41 433; Cyclopropancarbonsäure-N-alkoxyphenoxyalkylamide: EP-A 285 934; Cyclopropancarbonsäure-N-6-Ringhetaryloxyphenoxyalkylamide: EP-A 351 617; Cyclopropanthiocarbonsäureamide: deutsche Anmeldung 39 26 468), deren Wirkung gegen Schädlinge beispielsweise bei niedrigen Aufwandmengen nicht immer befriedigt. Aus EP-A-353 571 sind weiter phenoxyalkylsubstituierte Heteroaromaten bekannt.

Aufgabe der vorliegenden Erfindung waren neue, zur Schädlingsbekämpfung geeignete Verbindungen und Verfahren zu ihrer Herstellung und ihrer Verwendung.

Demgemäß wurden die eingangs definierten Cyclopropancarbonsäureamide und -thioamide I gefunden. Außerdem wurden Verfahren und neue Zwischenprodukte zur Herstellung dieser Cyclopropancarbonsäureamide und -thioamide, sie enthaltende Schädlingsbekämpfungsmittel und Verfahren zu ihrer Verwendung gefunden.

Die Cyclopropancarbonsäureamide und -thioamide I sind auf verschiedenen Wegen zugänglich.

Besonders vorteilhaft erhält man Verbindungen der allgemeinen Formel I, in denen Y Sauerstoff bedeutet, indem man einen 4-Hydroxyphenolether der allgemeinen Formel II in an sich bekannter Weise in einem inerten organischen Lösungsmittel in Gegenwart einer Base mit einem Nitril der allgemeinen Formel III, zu einem Hydrochinondiether der allgemeinen Formel IV verethert, die Nitrilfunktion anschließend in an sich bekannter Weise reduziert, das so erhaltene primäre Amin der allgemeinen Formel V danach mit einer aktivierten Cyclopropancarbonsäure der allgemeinen Formel VI in das entsprechende Amid der allgemeinen Formel VII überführt, aus VII durch Abspaltung der Schutzgruppe R das entsprechende Phenol der allgemeinen Formel VIII freisetzt welches dann in an sich bekannter Weise mit einer Heteroarylalkylverbindung der allgemeinen Formel IX, zu I verethert wird.

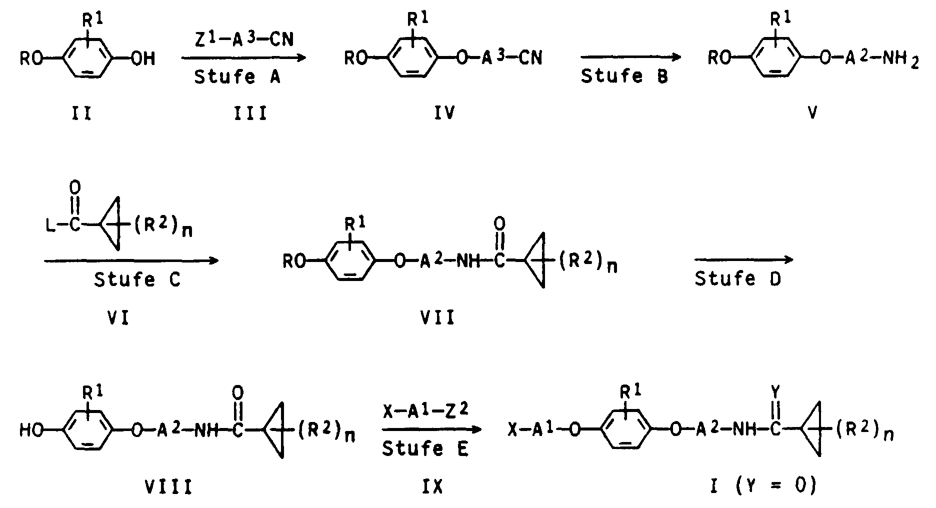

In den Formeln II, IV, V und VII bedeutet R eine unter den Umsetzungsbedingungen inerte Schutzgruppe z.B. C₁-C₆-Alkyl wie Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, vorzugsweise C₁-C₄-Alkyl wie Methyl, Ethyl, Propyl, 1-Methylethyl, Butyl, 1-Methylpropyl, 2-Methylpropyl und 1,1-Dimethylethyl, insbesondere 1,1-Dimethylethyl;

Benzyl, welches am Phenylring ein bis fünf Halogenatome wie Fluor, Chlor, Brom und Jod, Vorzugsweise Fluor, Chlor und Brom, und/oder ein bis drei der folgenden Reste tragen kann: C₁-C₄-Alkyl wie Methyl, Ethyl, Propyl, 1-Methylethyl, Butyl, 1-Methylpropyl, 2-Methylpropyl und 1,1-Dimethylethyl, vorzugsweise Methyl, Ethyl, Propyl, 1-Methylethyl und 1,1-Dimethylethyl, insbesondere Methyl, Ethyl und 1,1-Dimethylethyl; C₁-C₄-Alkoxy wie Methoxy, Ethoxy, Propyloxy, 1-Methylethoxy, Butyloxy, 1-Methyl-propyloxy, 2-Methylpropyloxy und 1,1-Dimethylethoxy;

dreifach durch C₁-C₆-Alkylgruppen und/oder unsubstituiertes oder substituiertes Phenyl wie vorstehend ausgeführt, substituiertes Silyl, insbesondere Trimethylsilyl, tert.-Butyl-dimethylsilyl, Phenyl-dimethylsilyl und tert.-Butyl-diphenylsilyl,

C₁-C₄-Alkoxy-C₁-C₄-alkyl wie in 1- oder 2-Position durch Methoxy, Ethoxy, Propyloxy, 1-Methylethoxy, Butyloxy, 1-Methyl-propyloxy, 2-Methylpropyloxy und 1,1-Dimethylethoxy substituiertes Methyl, Ethyl, Propyl, 1-Methylethyl, Butyl, 1-Methylpropyl, 2-Methylpropyl und 1,1-Dimethylethyl, insbesondere Methoxymethyl, oder

C₁-C₄-Alkoxy-ethoxy-C₁-C₄-alkyl wie in 1- oder 2-Position durch Methoxyethoxy, Ethoxyethoxy, Propyloxyethoxy, 1-Methylethoxyethoxy, Butyloxyethoxy, 1-Methyl-propyloxyethoxy, 2-Methylpropyloxyethoxy und 1,1-Dimethylethoxyethoxy substituiertes Methyl, Ethyl, Propyl, 1-Methylethyl, Butyl, 1-Methylpropyl, 2-Methylpropyl und 1,1-Dimethylethyl, insbesondere Methoxyethoxymethyl.

Z¹ in der Formel III und Z² in der Formel IX stehen unabhängig voneinander für nukleofuge Abgangsgruppen, z.B. Halogen wie insbesondere Chlor, Brom und Jod, C₁-C₄-Alkylsulfonyl wie Methylsulfonyl, Ethylsulfonyl, Propylsulfonyl, 1-Methylethylsulfonyl, Butylsulfonyl, 1-Methylpropylsulfonyl, 2-Methylpropylsulfonyl und 1,1-Dimethylethylsulfonyl, insbesondere Methylsulfonyl;

Phenylsulfonyl, welches am Phenylring ein bis fünf Halogenatome wie Fluor, Chlor, Brom und Jod, vorzugsweise Fluor, Chlor und Brom, und/oder ein bis drei der folgenden Reste tragen kann: C₁-C₄-Alkyl wie Methyl, Ethyl, Propyl, 1-Methylethyl, Butyl, 1-Methylpropyl, 2-Methylpropyl und 1,1-Dimethylethyl, vorzugsweise Methyl, Ethyl, Propyl, 1-Methylethyl und 1,1-Dimethylethyl, insbesondere Methyl, Ethyl und 1,1-Dimethylethyl;

A³ in der Formel III bedeutet Methylen oder Ethylen, welches ein oder zwei C₁-C₃-Alkylreste wie Methyl, Ethyl, Propyl und 1-Methylethyl, vorzugsweise Methyl und Ethyl, insbesondere Methyl tragen kann.

In der Formel VI steht L für eine nukleophil verdrängbare Abgangsgruppe wie Halogen, beispielsweise Chlor, Brom und Jod; C₁-C₄-Alkyloxy wie Methyloxy, Ethyloxy, Propyloxy, 1-Methylethyloxy, Butyloxy, 1-Methylpropyloxy, 2-Methylpropyloxy und 1,1-Dimethylethyloxy, insbesondere Methyloxy und Ethyloxy.

Im einzelnen werden die Umsetzungen wie folgt durchgeführt:

### Reaktionsstufe A: Veretherung

(Houben-Weyl, Methoden der organischen Chemie, Bd. VI/3, S. 1ff, S. 49ff, Thieme Verlag, Stuttgart, 1965) 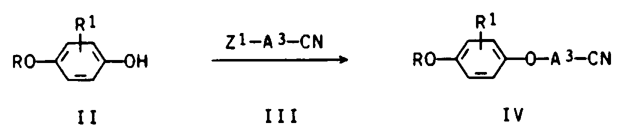 Üblicherweise erfolgt die Veretherung bei Temperaturen von -20 bis 170°C, vorzugsweise von 20 bis 130°C.

Die Veretherung verläuft im allgemeinen oberhalb von 20°C mit ausreichender Geschwindigkeit. 130°C müssen in der Regel für einen vollständigen Umsatz nicht überschritten werden. Die Reaktion kann unter Wärmeentwicklung verlaufen, weshalb es vorteilhaft sein kann, eine Kühlmöglichkeit vorzusehen.

Als Basen kommen allgemein anorganische Verbindungen wie Alkalimetall-und Erdalkalimetallhydroxide, beispielsweise Lithiumhydroxid, Natriumhydroxid, Kaliumhydroxid und Calziumhydroxid, Alkalimetall- und Erdalkalimetalloxide, beispielsweise Lithiumoxid, Natriumoxid, Calziumoxid und Magnesiumoxid, Alkalimetall- und Erdalkalimetallhydride wie Lithiumhydrid, Natriumhydrid, Kaliumhydrid und Calziumhydrid, Alkalimetall- und Erdalkalimetallcarbonate, beispielsweise Lithiumcarbonat, Natriumcarbonat, Kaliumcarbonat und Calziumcarbonat, Alkalimetallhydrogencarbonate, beispielsweise Natriumhydrogencarbonat, metallorganische Verbindungen, insbesondere Alkalimetallalkyle, beispielsweise Methyllithium, Butyllithium und Phenyllithium, Alkalimetall- und Erdalkalimetallalkoholate, beispielsweise Natriummethanolat, Natriumethanolat, Kaliumethanolat, Kalium-tert.-butanolat und Dimethoxymagnesium, außerdem organische Basen, z.B. tertiäre Amine wie Trimethylamin, Triethylamin, Diisopropylethylamin und N-Methylpiperidin, Pyridin, substituierte Pyridine wie Collidin, Lutidin und 4-Dimethylaminopyridin sowie bicyclische Amine in Betracht. Besonders bevorzugt werden Kaliumhydroxid, Kaliumcarbonat, Natriummethylat, Natriumethylat, Kalium-tert.-butylat und Natriumhydrid. Man verwendet im Normalfall mindestens äquivalente Mengen der Base, kann diese aber auch im überschuß oder gegebenenfalls als Lösungsmittel verwenden.

Als Lösungsmittel eignen sich beispielsweise aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Ethylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol und Isopropanol, sowie aprotisch dipolare Lösungsmittel wie Dimethylsulfoxid, Dimethylformamid und Pyridin besonders bevorzugt Acetonitril, Ethanol und Dimethylformamid.

Es können auch Gemische der genannten Lösungsmittel verwendet werden.

Die Ausgangsstoffe werden üblicherweise in stöchiometrischen Mengen miteinander umgesetzt. Es kann, beispielsweise zur Steigerung der Ausbeute, vorteilhaft sein, einen der Ausgangsstoffe in einem Überschuß von 0,1 bis 10 mol-Äq., vorzugsweise 0,2 bis 1,5 mol-Äq. zu verwenden.

Sofern Z¹ bzw. Z² in einer der Formeln III bzw. IX für Chlor oder Brom steht, kann die Umsetzung im allgemeinen durch Zugabe von katalytischen Mengen an Kaliumiodid beschleunigt werden. Der Katalysator wird üblicherweise in Mengen von 5 bis 20 mol-% verwendet.

### Reaktionsstufe B: Reduktion

(Organikum, 15. Auflage, S. 540ff, S. 614ff, VEB Deutscher Verlag der Wissenschaften, Berlin 1977) 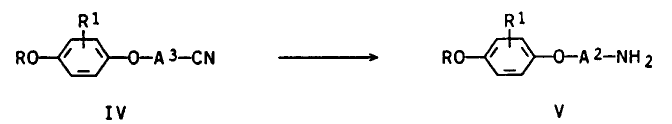 üblicherweise erfolgt die Reduktion bei Temperaturen von -30 bis 100°C, vorzugsweise von 0 bis 80°C.

Die Reduktion verläuft im allgemeinen oberhalb von 0°C mit ausreichender Geschwindigkeit. 80°C müssen in der Regel für einen vollständigen Umsatz nicht überschritten werden.

Als Reduktionsmittel kommen allgemein komplexe Hydride wie Lithiumaluminiumhydrid und Borhydrid oder elementarer Wasserstoff in Gegenwart von Metallkatalysatoren wie Raney-Nickel in Betracht. Besonders bevorzugt werden Lithiumaluminiumhydrid und Wasserstoff/Raney-Nickel.

Man verwendet üblicherweise mindestens äquivalente Mengen des Reduktionsmittels, kann dieses aber auch im Überschuß verwenden.

Als Lösungsmittel eignen sich beispielsweise aliphatische Kohlenwasserstoffe wie Pentan, Hexan, Cyclohexan und Petrolether, aromatische Kohlenwasserstoffe wie Toluol, o-, m- und p-Xylol, Ether wie Diethylether, Diisopropylether, tert.-Butylmethylether, Dioxan, Anisol und Tetrahydrofuran, Alkohole wie Methanol, Ethanol und Isopropanol, besonders bevorzugt Diethylether und Tetrahydrofuran.

Es können auch Gemische der genannten Lösungsmittel verwendet werden.

### Reaktionsstufe C: Amid-Bildung

(Organikum, 15. Auflage, S. 511ff, VEB Deutscher Verlag der Wissenschaften, Berlin 1977) 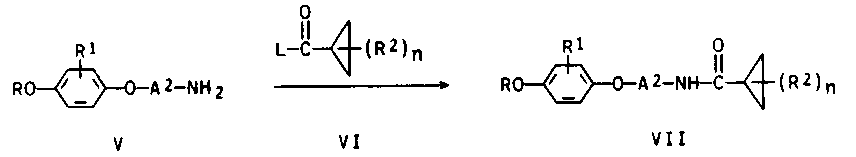 Üblicherweise erfolgt die Amid-Bildung bei Temperaturen von -20 bis 100°C, vorzugsweise von 0 bis 80°C.

Die Reaktion verläuft im allgemeinen oberhalb von 0°C mit ausreichender Geschwindigkeit. 80°C müssen in der Regel für einen vollständigen Umsatz nicht überschritten werden.

Als Lösungsmittel eignen sich beispielsweise aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Ethylenchlorid, Chloroform und Chlorbenzol, Ether wie Diethylether, Diisopropylether, tert.-Butylmethylether, Dioxan, Anisol und Tetrahydrofuran, Alkohole wie Methanol, Ethanol und Isopropanol, sowie aprotisch dipolare Lösungsmittel wie Pyridin, besonders bevorzugt Diethylether, Tetrahydrofuran und Methylenchlorid.

Es können auch Gemische der genannten Lösungsmittel verwendet werden.

### Reaktionsstufe D: Etherspaltung

(Houben-Weyl, Methoden der organischen Chemie, Bd. VI/1c, S. 314ff, Thieme Verlag, Stuttgart, 1976) 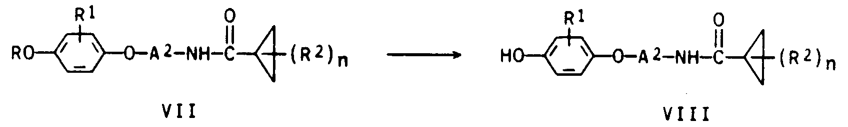 Die Abspaltung der Schutzgruppe R aus der Verbindung VII erfolgt in ebenfalls bekannter Weise in einem Lösungsmittel in Gegenwart einer Säure oder eines sauren Katalysators.

Die Ether VII werden im allgemeinen bei Temperaturen von -20 bis 120°C, vorzugsweise 20 bis 100°C gespalten.

Als Lösungsmittel für die Spaltung eignen sich die im Vorstehenden für die Veretherung genannten, insbesondere Methanol, Ethanol, Chloroform und Dioxan und entsprechende Gemische.

Als Säuren und saure Katalysatoren finden anorganische Säuren wie Fluorwasserstoffsäure, Salzsäure, Bromwasserstoffsäure, Schwefelsäure und Perchlorsäure, Lewis-Säuren wie Bortrifluorid, Aluminiumtrichlorid, Eisen-III-chlorid, Zinn, IV-chlorid, Titan-IV-chlorid und Zink-II-chlorid, sowie organische Säuren wie Ameisensäure, Essigsäure, Propionsäure, Oxalsäure, Zitronensäure und Trifluoressigsäure Verwendung. Die Säuren werden im allgemeinen in katalytischen Mengen eingesetzt, sie können aber auch äquimolar, im Überschuß oder gegebenenfalls als Lösungsmittel verwendet werden.

### Reaktionsstufe E: Veretherung

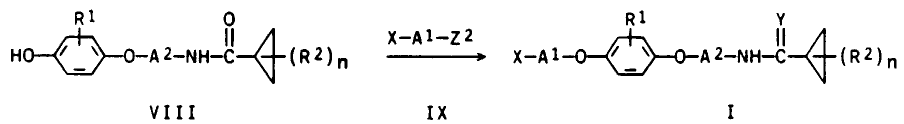

Die Veretherung der Verbindungen VIII zu den Produkten 1 wird im allgemeinen und im besonderen gemäß den bei Reaktionsstufe A beschriebenen Bedingungen durchgeführt.

Die Verbindungen der allgemeinen Formel I, in denen Y Schwefel bedeutet erhält man vorteilhaft dadurch, daß man ein Cyclopropancarbonsäureamid der allgemeinen Formel I, in der Y Sauerstoff bedeutet in an sich bekannter Weise in einem inerten organischen Lösungsmittel in Gegenwart eines Schwefel übertragenden Reagens umsetzt.

Als Schwefel übertragendes Reagens eignen sich P₄S₁₀ oder Lawesson-Reagenz [2,4-Bis-(4-methoxyphenyl)-1,3-dithia-2,4-diphosphetan-2,4-disulfid]. Solche Umsetzungen sind beschrieben in Houben-Weyl, Methoden der organischen Chemie, Bd. E 5, S. 1242ff, Thieme Verlag, Stuttgart 1985. Die Schwefelungen werden im allgemeinen bei Temperaturen von 0 bis 150°C, vorzugsweise 50 bis 120°C durchgeführt.

Als Lösungsmittel eignen sich beispielsweise aliphatische Kohlenwasserstoffe wie Petrolether, aromatische Kohlenwasserstoffe wie Toluol und Xylole, halogenierte Kohlenwasserstoffe, Ether wie Dioxan, Anisol, Glykoldimethylether und Diglykoldimethylether, und aromatische Amine wie Pyridin, insbesondere Toluol, Glykoldimethylether und Pyridin.

Man erhält die Verbindungen I beispielsweise auch dadurch, daß man ein Amin der allgemeinen Formel X, in an sich bekannter Weise in einem inerten organischen Lösungsmittel mit einer aktivierten Cyclopropancarbonsäure bzw. -thiocarbonsäure der allgemeinen Formel VI' umsetzt.

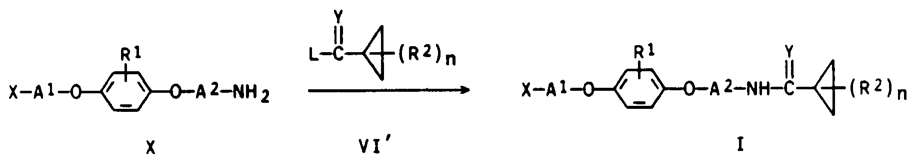

In der Formel VI' steht L für eine nukleophil verdrängbare Abgangsgruppe. In Fällen, wo Y Sauerstoff bedeutet, eignet sich als nucleophile Abgangsgruppe besonders Halogen wie Fluor, Chlor, Brom und Jod, vorzugsweise Chlor und Brom und Alkoxy, vorzugsweise C₁-C₄-Alkoxy wie Methoxy, Ethoxy, Propyloxy, 1-Methylethoxy, Butyloxy, 1-Methyl-propyloxy, 2-Methylpropyloxy und 1,1-Dimethylethoxy, insbesondere Methoxy und Ethoxy.

In Fällen, wo Y Schwefel bedeutet, eignet sich als nucleophile Abgangsgruppe besonders Alkylthio, vorzugsweise C1-C4-Alkylthio wie Methylthio, Ethylthio, Propylthio, 1-Methylethylthio, Butylthio, 1-Methyl-propylthio, 2-Methylpropylthio und 1,1-Dimethylethylthio, insbesondere Methylthio und Ethylthio.

Üblicherweise erfolgt die Amid-Bildung bei Temperaturen von -50 bis 150°C, vorzugsweise von -20 bis 120°C.

Die Amid-Bildung verläuft im allgemeinen oberhalb von -20°C mit ausreichender Geschwindigkeit. 120°C müssen in der Regel für einen vollständigen Umsatz nicht überschritten werden. Die Reaktion kann unter Wärmeentwicklung verlaufen, weshalb es vorteilhaft sein kann, eine Kühlmöglichkeit vorzusehen.

Als Lösungsmittel eignen sich beispielsweise aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Ethylenchlorid, Chloroform und Chlorbenzol, Ether wie Diethylether, Diisopropylether, tert.-Butylmethylether, Dioxan, Anisol und Tetrahydrofuran, Alkohole wie Methanol, Ethanol und Isopropanol, sowie aprotisch dipolare Lösungsmittel wie Pyridin, besonders bevorzugt Diethylether, Tetrahydrofuran und Methylenchlorid.

Es können auch Gemische der genannten Lösungsmittel verwendet werden.

Die Ausgangsstoffe werden üblicherweise in stöchiometrischen Mengen miteinander umgesetzt. Es kann, beispielsweise zur Steigerung der Ausbeute, vorteilhaft sein, einen der Ausgangsstoffe in einem Überschuß von 0,1 bis 10 mol-Äq., vorzugsweise 0,2 bis 1,5 mol-Äq. zu verwenden.

Die für dieses Verfahren benötigten Amine der allgemeinen Formel X erhält man durch Umsetzung der 4-Hydroxyphenolether der allgemeinen Formel II mit einer Heteroarylalkylverbindung der allgemeinen Formel IX zu einem Diether der allgemeinen Formel V', Abspaltung der Schutzgruppe, Veretherung des so erhaltenen entsprechenden Phenols der allgemeinen Formel VIII' mit einem Nitril der allgemeinen Formel III zu einem Hydrochinondiether der allgemeinen Formel IV' und anschließende Reduktion der Nitrilfunktion.

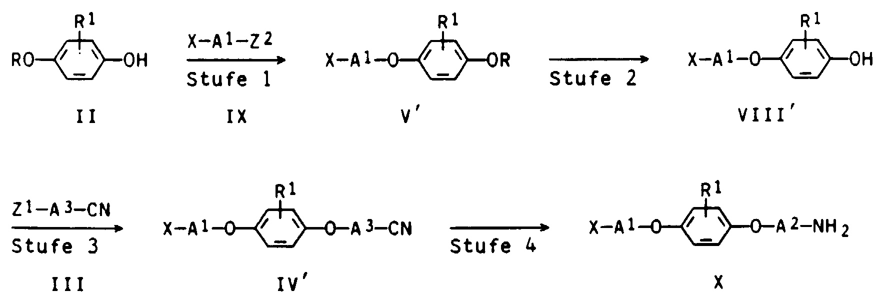

### Reaktionsstufe 1: Veretherung

Die Veretherung der Verbindungen II zu den Diethern der allgemeinen Formel V' wird im allgemeinen und im besonderen gemäß den bei Reaktionsstufe A beschriebenen Bedingungen durchgeführt.

### Reaktionsstufe 2: Etherspaltung

Die Etherspaltung der Verbindungen V' zu den Phenolen der allgemeinen Formel VIII' wird im allgemeinen und im besonderen gemäß den bei Reaktionsstufe D beschriebenen Bedingungen durchgeführt.

### Reaktionsstufe 3: Veretherung

Die Veretherung der Verbindungen VIII' zu den Diethern der allgemeinen Formel IV' wird im allgemeinen und im besonderen gemäß den bei Reaktionsstufe A beschriebenen Bedingungen durchgeführt.

### Reaktionsstufe 4: Reduktion

Die Reduktion der Verbindungen IV' zu den Aminen X wird im allgemeinen und im besonderen gemäß den bei Reaktionsstufe B beschriebenen Bedingungen durchgeführt.

Die Reaktionsgemische werden in üblicher Weise aufgearbeitet, z.B. durch Mischen mit Wasser, Trennung der Phasen und gegebenenfalls chromatographische Reinigung der Rohprodukte. Die Zwischen- und Endprodukte fallen z.T. in Form farbloser oder schwach bräunlicher, zäher Öle an, die unter vermindertem Druck und bei mäßig erhöhter Temperatur von flüchtigen Anteilen befreit oder gereinigt werden. Sofern die Zwischen- und Endprodukte als Feststoffe erhalten werden, kann die Reinigung auch durch Umkristallisieren oder Digerieren erfolgen.

Die für die Umsetzung benötigten Hetarylalkylderivate der Formel IX sind entweder bekannt, kommerziell erhältlich oder lassen sich nach allgemein bekannten Verfahren herstellen.

Verfahren zur Herstellung von Thiophenderivaten finden sich beispielsweise in: Comprehensive Heterocyclic Chemistry, A.R. Katritzky und C.W. Rees, Vol.4, S. 863ff, Pergamon Press 1984, Thiazolderivate, Oxazolderivate, Isothiazolderivate, Thiadiazolderivate und Oxadiazolderivate z.B. in Comprehensive Heterocyclic Chemistry, A.R. Katritzky und C.W. Rees, Vol. 6, S. 131, 177, 235, 365, 427, 545ff, Pergamon Press 1984; Imidazolderivate z.B. in: Advances in Heterocyclic Chemistry, Vol. 27, S. 242ff, 1980, Pyrazol- und Triazolderivate z.B. in Comprehensive Heterocyclic Chemistry, A.R. Katritzky und C.W. Rees, Vol. 5, S. 167, 733ff, Pergamon Press 1984; Isoxazolderivate z.B. in DE-A-25 49 962 und DE-A-27 54 832.

N-Methylazole sind zum Teil aus Heterocycles 24, 2233-2237 (1986) bekannt oder können nach der dort beschriebenen Methode durch Umsetzung des Azols mit para-Formaldehyd erhalten werden.

N-Hydroxypyrazole sind beispielsweise in der DE-A 38 20 738 und in der DE-A 38 20 739 und N-Hydroxytriazole sind beispielsweise in der DE-A 39 00 347 beschrieben oder können nach den dort beschriebenen Verfahren hergestellt werden.

Im Hinblick auf die bestimmungsgemäße Verwendung der Cyclopropancarbonsäureamide und -thioamide I in Schädlingsbekämpfungsmitteln kommen als Index und Substituenten die folgenden in Betracht:
- n: 0, 1 oder 2, wobei die Reste R² verschieden sein können, wenn n für 2 steht, vorzugsweise 0.
- A¹: Methylen, Ethylen oder Propylen, vorzugsweise Methylen oder Ethylen, insbesondere Methylen, wobei diese Gruppen ein oder zwei C₁-C₃-Alkylreste wie Methyl, Ethyl, Propyl und 1-Methylethyl, vorzugsweise Methyl und Ethyl, insbesondere Methyl tragen können;
- A²: Ethylen oder Propylen, wobei diese Gruppen ein oder zwei C₁-C₃-Alkylreste wie Methyl, Ethyl, Propyl und 1-Methylethyl, vorzugsweise Methyl und Ethyl, insbesondere Methyl tragen können;
- R¹: Wasserstoff,
Halogen wie Fluor, Chlor, Brom und Iod, vorzugsweise Fluor, Chlor und Brom, insbesondere Fluor und Chlor, oder C₁-C₆-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, vorzugsweise C₁-C₃-Alkyl, insbesondere Methyl und Ethyl;
- R²: Halogen wie Fluor, Chlor, Brom und Iod, vorzugsweise Fluor, Chlor und Brom, insbesondere Fluor und Chlor,
oder C₁-C₃-Alkyl wie Methyl, Ethyl, Propyl und 1-Methylethyl, vorzugsweise Methyl und Ethyl insbesondere Methyl;
- X: ein über ein C-Atom oder ein N-Atom gebundener 5-gliedriger heteroaromatischer Rest, enthaltend ein bis drei Stickstoffatome oder ein Sauerstoff- oder ein Schwefelatom oder enthaltend ein oder zwei Stickstoffatome und ein Sauerstoff- oder ein Schwefelatom als Heteroatome, wie 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 1-Pyrazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 1-Imidazolyl, 1,2,4-Oxadiazol-3-yl, -3-yl, 1,2,4-Oxadiazol-5-yl, 1,2,4-Thiadiazol-3-yl, 1,2,4-Thiadiazol-5-yl, 1,2,4-Triazol-3-yl, 1,2,4-Triazol-1-yl, 1,2,4-Triazol-4-yl, 1,3,4-Oxadiazol-2-yl, -2-yl, 1,3,4-Thiadiazol-2-yl und 1,3,4-Triazol-2-yl, vorzugsweise ein Heteroaromat enthaltend ein bis zwei Stickstoffatome und/oder ein Sauerstoff- oder Schwefelatom als Heteroatome, insbesondere 1-Imidazolyl, 3-Isoxazolyl, 5-Isoxazolyl, 1,3,4-Oxadiazol-2-yl und 1,3,4-Thiadiazol-2-yl;
oder ein Pyrazolyl-1-oxy- oder Triazolyl-1-oxyrest, wobei diese 5-Ring Heteroaromaten an ihren Kohlenstoffringgliedern ein bis drei der folgenden Reste tragen können: Nitro, Cyano, Halogen wie Fluor, Chlor, Brom und Jod, vorzugsweise Fluor, Chlor und Brom, insbesondere Fluor und Chlor;

C₁-C₆-Alkyl wie Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Oimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, vorzugsweise C₁-C₄-Alkyl, insbesondere Methyl, Ethyl und Propyl;
C₁-C₄-Halogenalkyl, besonders C₁-C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl, insbesondere Trifluormethyl;
C₁-C₆-Alkoxy wie Methyloxy, Ethyloxy, Propyloxy, 1-Methylethyloxy, Butyloxy, 1-Methyl-propyloxy, 2-Methylpropyloxy, 1,1-Dimethylethyloxy, Pentyloxy, 1-Methylbutyloxy, 2-Methylbutyloxy, 3-Methylbutyloxy, 1,1-Dimethylpropyloxy, 1,2-Dimethylpropyloxy, 2,2-Dimethylpropyloxy, 1-Ethylpropyloxy, Hexyloxy, 1-Methylpentyloxy, 2-Methylpentyloxy, 3-Methylpentyloxy, 4-Methylpentyloxy, 1,1-Dimethylbutyloxy, 1,2-Dimethylbutyloxy, 1,3-Dimethylbutyloxy, 2,2-Dimethylbutyloxy, 2,3-Dimethylbutyloxy, 3,3-Dimethylbutyloxy, 1-Ethylbutyloxy, 2-Ethylbutyloxy, 1,1,2-Trimethylpropyloxy, 1,2,2-Trimethylpropyloxy, 1-Ethyl-1-methylpropyloxy und 1-Ethyl-2-methylpropyloxy, vorzugsweise C₁-C₃-Alkoxy, insbesondere Methoxy und Ethoxy;
C₁-C₄-Halogenalkoxy, besonders C₁-C₂-Halogenalkyloxy wie Chlormethyloxy, Dichlormethyloxy, Trichlormethyloxy, Fluormethyloxy, Difluormethyloxy, Trifluormethyloxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy, 2,2,2-Trichlorethyloxy und Pentafluorethyloxy, insbesondere Trifluormethoxy und Difluormethoxy;
C₁-C₄-Alkylthio wie Methylthio, Ethylthio, Propylthio, 1-Methylethylthio, Butylthio, 1-Methyl-propylthio, 2-Methylpropylthio und 1,1-Dimethylethylthio, vorzugsweise C₁-C₂-Alkylthio, insbesondere Methylthio;
C₁-C₄-Halogenalkylthio, besonders C₁-C₂-Halogenalkylthio wie Chlormethylthio, Dichlormethylthio, Trichlormethylthio, Fluormethylthio, Difluormethylthio, Trifluormethylthio, Chlorfluormethylthio, Dichlorfluormethylthio, Chlordifluormethylthio, 1-Fluorethylthio, 2-Fluorethylthio, 2,2-Difluorethylthio, 2,2,2-Trifluorethylthio, 2-Chlor-2-fluorethylthio, 2-Chlor-2,2-difluorethylthio, 2,2-Dichlor-2-fluorethylthio, 2,2,2-Trichlorethylthio und Pentafluorethylthio, insbesondere Trifluormethylthio;
durch C₁-C₄-Alkoxy wie vorstehend genannt vorzugsweise in 1- oder 2-Position substituiertes C₁-C₄-Alkyl wie vorstehend genannt, z.B. Methoxymethyl, Ethoxymethyl, Propyloxymethyl, 1-Methylethoxymethyl, Methoxy-1-ethyl, Ethoxy-1-ethyl, Propyl-oxy-1-ethyl, 1-Methylethoxy-1-ethyl, Methoxy-2-ethyl, Ethoxy-2-ethyl, Propyloxy-2-ethyl, 1-Methylethoxy-2-ethyl, Methoxy-1-propyl, Ethoxy-1-propyl, Propyloxy-1-propyl, 1-Methylethoxy-1-propyl, Methoxy-2-propyl, Ethoxy-2-propyl, Propyloxy-2-propyl, 1-Methylethoxy-2-propyl, Methoxy-3-propyl, Ethoxy-3-propyl, Propyloxy-3-propyl, 1-Methylethoxy-3-propyl, vorzugsweise C₁-C₂-Alkoxy-C₁-C₂-alkyl insbesondere Methoxymethyl, Ethoxymethyl und Methoxyethyl;
C₃-C₈-Cycloalkyl wie Cyclopropyl, Cyclobutyl, Cyclopentyl, Cyclohexyl, Cycloheptyl und Cyclooctyl, insbesondere Cyclopropyl;
C₂-C₈-Alkenyl, besonders C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl- 1-Methyl-2-butenyl, 2-Methyl-1-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl,2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl,1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-l-methyl-2-propenyl, 1-Ethyl-2-methyl-1-propenyl und 1-Ethyl-2-methyl-2-propenyl, vorzugsweise C₂-C₄-Alkenyl insbesondere Ethenyl, 1-Propenyl und 2-Propenyl;
C₂-C₈-Alkinyl, besonders C₂-C₆-Alkinyl wie 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 1-Ethyl2-butinyl,1-Ethyl-3-butinyl, 2-Ethyl-3-butinyl und 1-Ethyl-1-methyl-2-propinyl, vorzugsweise C₂-C₃-Alkinyl, insbesondere Ethinyl und 1-Propinyl;
Aryl wie Phenyl, 1-Naphthyl und 2-Naphthyl, vorzugsweise Phenyl oder durch Aryl wie vorstehend genannt substituiertes C₁-C₁₀-Alkyl, besonders C₁-C₆-Alkyl wie vorstehend genannt, vorzugsweise C₁-C₄-Alkyl wie vorstehend genannt, insbesondere Methyl, Ethyl und Propyl, wobei die aromatischen Reste ihrerseits ein bis fünf Halogenatome wie Fluor, Chlor, Brom und Jod, vorzugsweise Fluor, Chlor und Brom, insbesondere Fluor und Chlor, und/oder ein bis drei der folgenden Gruppen tragen können: C₁-C₄-Alkyl wie Methyl, Ethyl, Propyl, 1-Methylethyl, Butyl, 1-Methyl-propyl, 2-Methylpropyl und 1,1-Dimethylethyl, vorzugsweise C₁-C₃-Alkyl, insbesondere Methyl und Ethyl;
C₁-C₄-Halogenalkyl, besonders C₁-C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl, insbesondere Trifluormethyl;
C₁-C₄-Alkoxy wie Methyloxy, Ethyloxy, Propyloxy, 1-Methylethyloxy, Butyloxy, 1-Methyl-propyloxy, 2-Methylpropyloxy und 1,1-Dimethylethyloxy, vorzugsweise C₁-C₃-Alkoxy, insbesondere Methoxy und Ethoxy;
C₁-C₄-Halogenalkoxy, besonders C₁-C₂-Halogenalkyloxy wie Chlormethyloxy, Dichlormethyloxy, Trichlormethyloxy, Fluormethyloxy, Difluormethyloxy, Trifluormethyloxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy, 2,2,2-Trichlorethyloxy und Pentafluorethyloxy, insbesondere Difluormethoxy und Trifluormethoxy;
C₁-C₄-Alkylthio wie Methylthio, Ethylthio, Propylthio, 1-Methylethylthio, Butylthio, 1-Methyl-propylthio, 2-Methylpropylthio und 1,1-Dimethylethylthio, vorzugsweise C₁-C₃-Alkylthio, insbesondere Methylthio und Ethylthio;
oder C₁-C₄-Halogenalkylthio besonders C₁-C₂-Halogenalkylthio wie Chlormethylthio, Dichlormethylthio, Trichlormethylthio, Fluormethylthio, Difluormethylthio, Trifluormethylthio, Chlorfluormethylthio, Dichlorfluormethylthio, Chlordifluormethylthio, 1-Fluorethylthio, 2-Fluorethylthio, 2,2-Difluorethylthio, 2,2,2-Trifluorethylthio, 2-Chlor-2-fluorethylthio, 2-Chlor-2,2-difluorethylthio, 2,2-Dichlor-2-fluorethylthio, 2,2,2-Trichlorethylthio und Pentafluorethylthio, insbesondere Trifluormethylthio;

- Y: Sauerstoff oder Schwefel.

Besonders bevorzugt sind Cyclopropancarbonsäureamide und -thioamide der allgemeinen Formel I, in denen der Substituent A¹ folgende Bedeutung hat: Methylen, Ethylen oder Propylen, wobei diese Gruppen ein oder zwei Methylgruppen tragen können.

Besonders bevorzugt sind auch Cyclopropancarbonsäureamide und -thioamide der allgemeinen Formel I, in denen der Substituent A² folgende Bedeutung hat: Ethylen oder Propylen, wobei diese Gruppen ein oder zwei Methylgruppen tragen können.

Daneben werden Cyclopropancarbonsäureamide und -thioamide der allgemeinen Formel I bevorzugt, in denen der Substituent R¹ folgende Bedeutung hat: Wasserstoff, Fluor, Chlor, Brom oder Methyl.

Ebenso werden Cyclopropancarbonsäureamide und -thioamide der allgemeinen Formel I bevorzugt, in denen der Substituent R² folgende Bedeutung hat: Fluor, Chlor und/oder Methyl.

Auperdem werden Cyclopropancarbonsäureamide und -thioamide der allgemeinen Formel I bevorzugt, in denen der Substituent X einen der folgenden Ringe X-1 bis X-32 repräsentiert. Die möglichen Substituenten an diesen Ringen wurden der übersichtlichkeit wegen nicht eingezeichnet; die Bindung zu A¹ ist als .- bezeichnet.

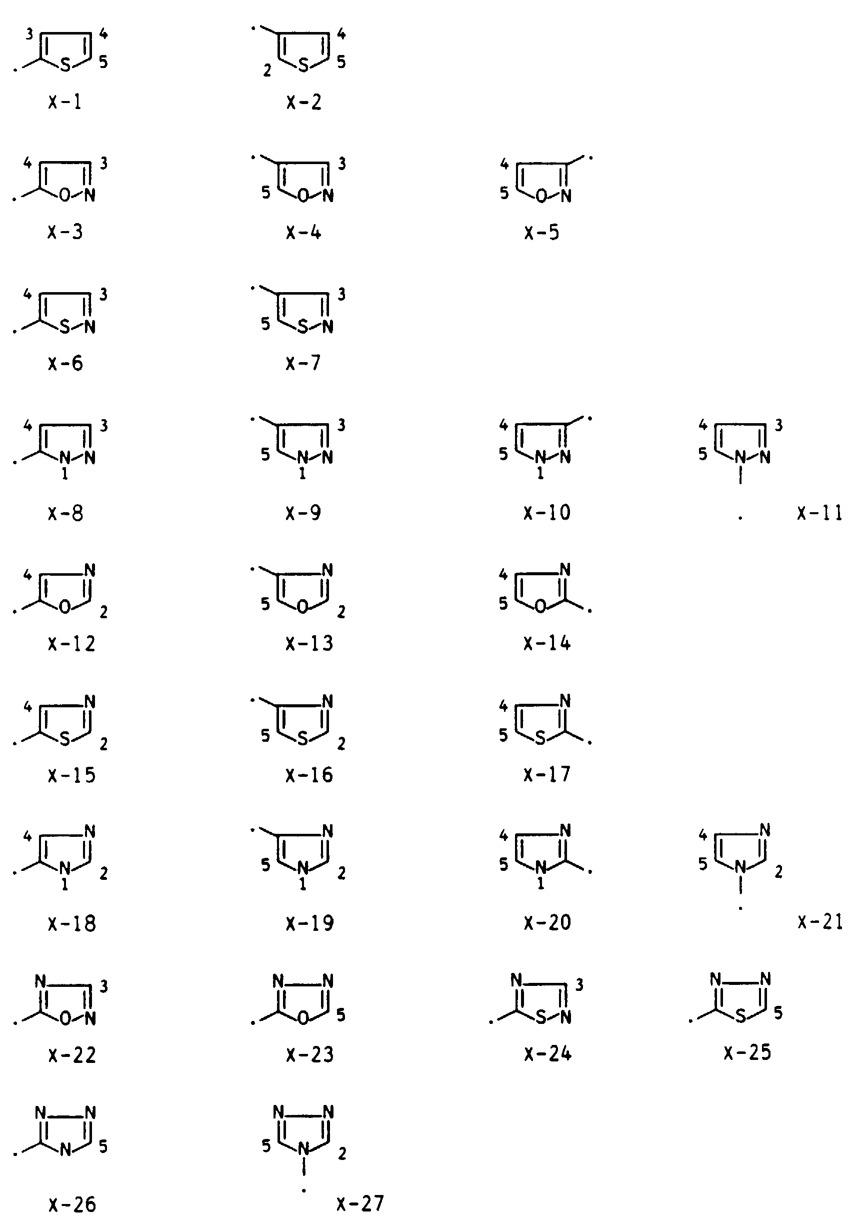

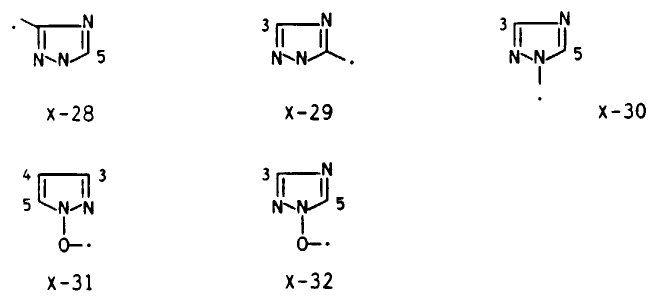

Beispiele für insbesondere bevorzugte Verbindungen der allgemeinen Formel I sind in der folgenden Tabelle zusammengestellt:

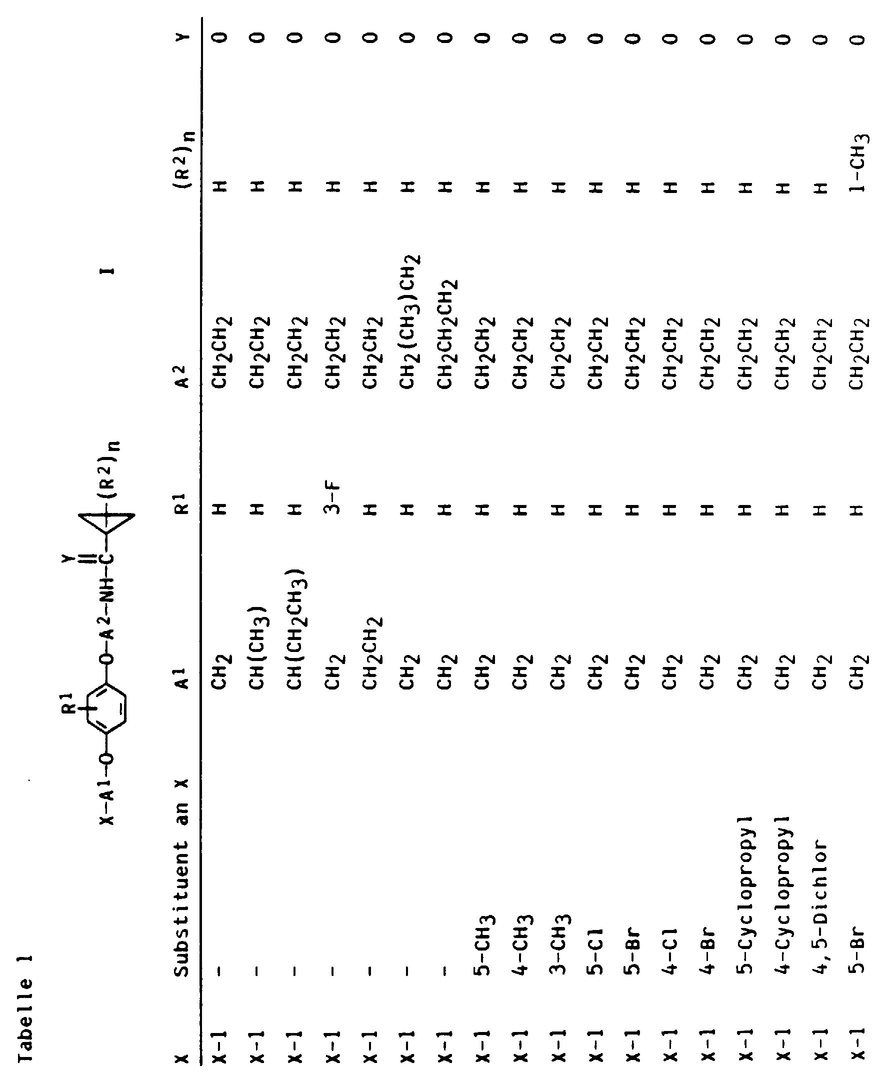

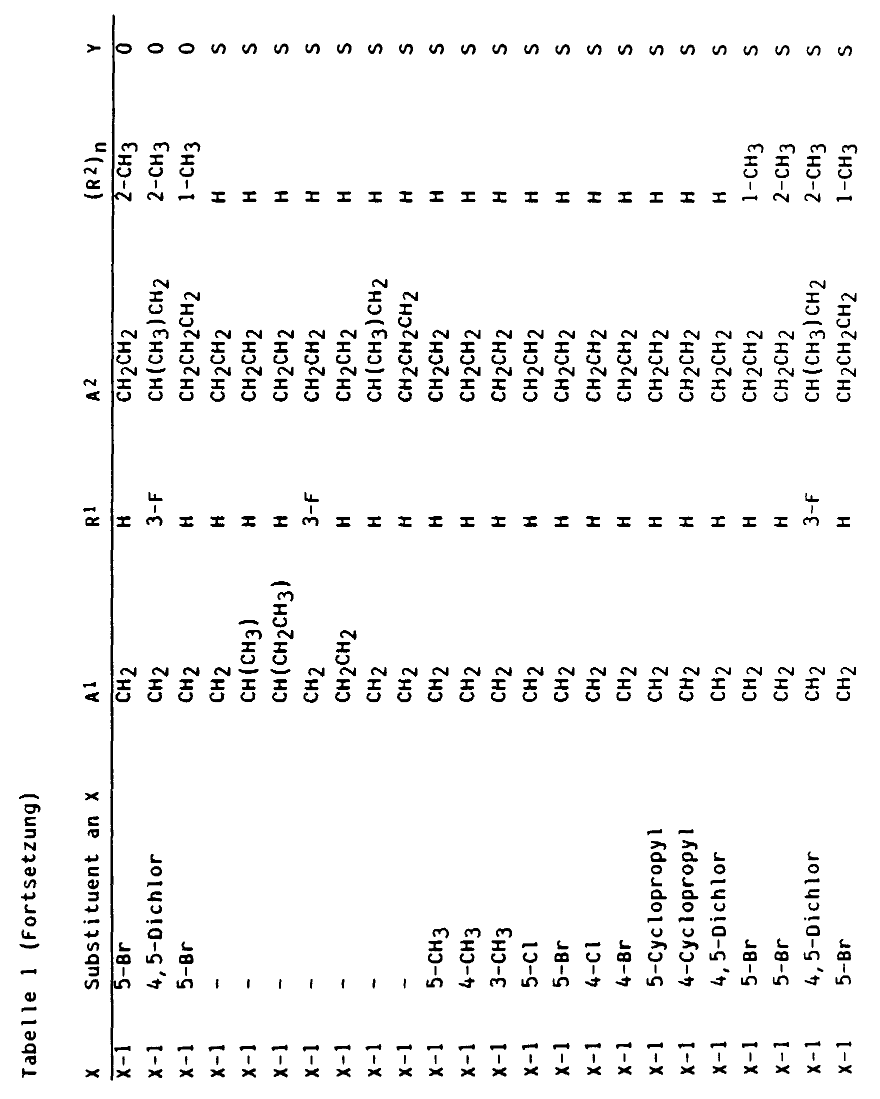

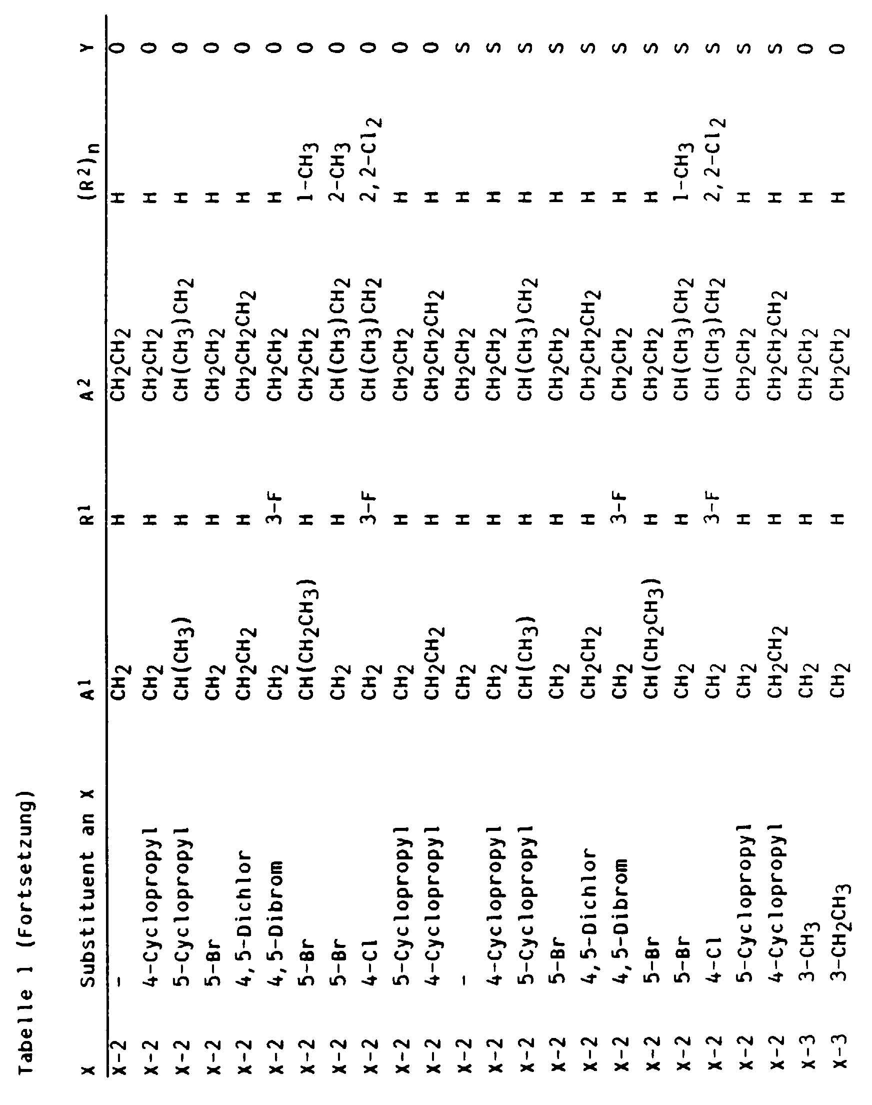

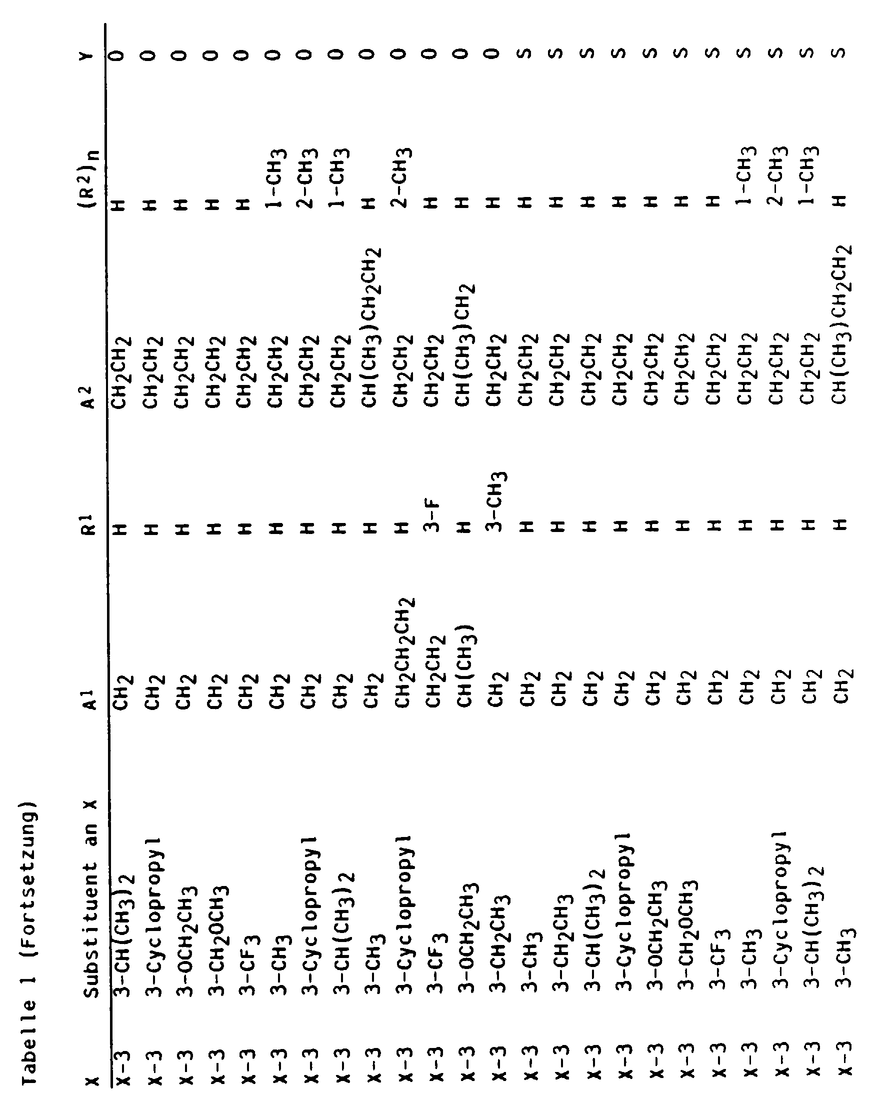

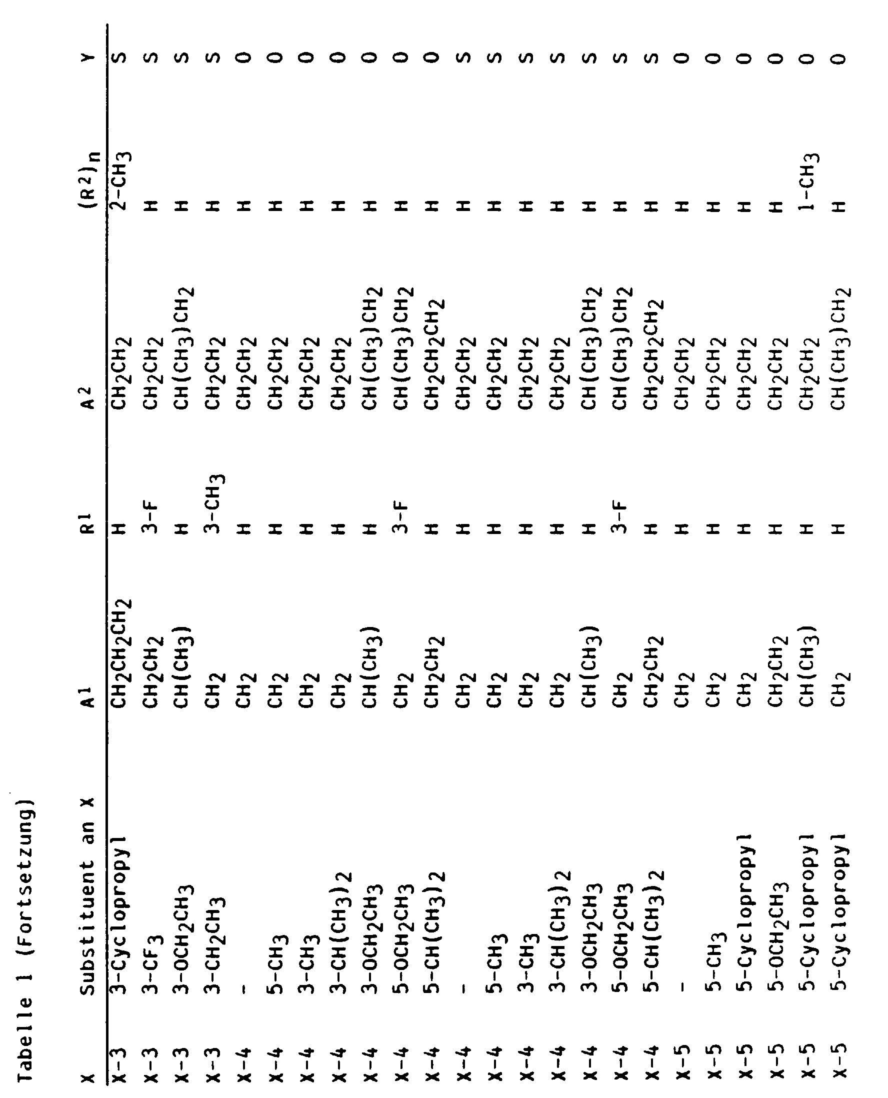

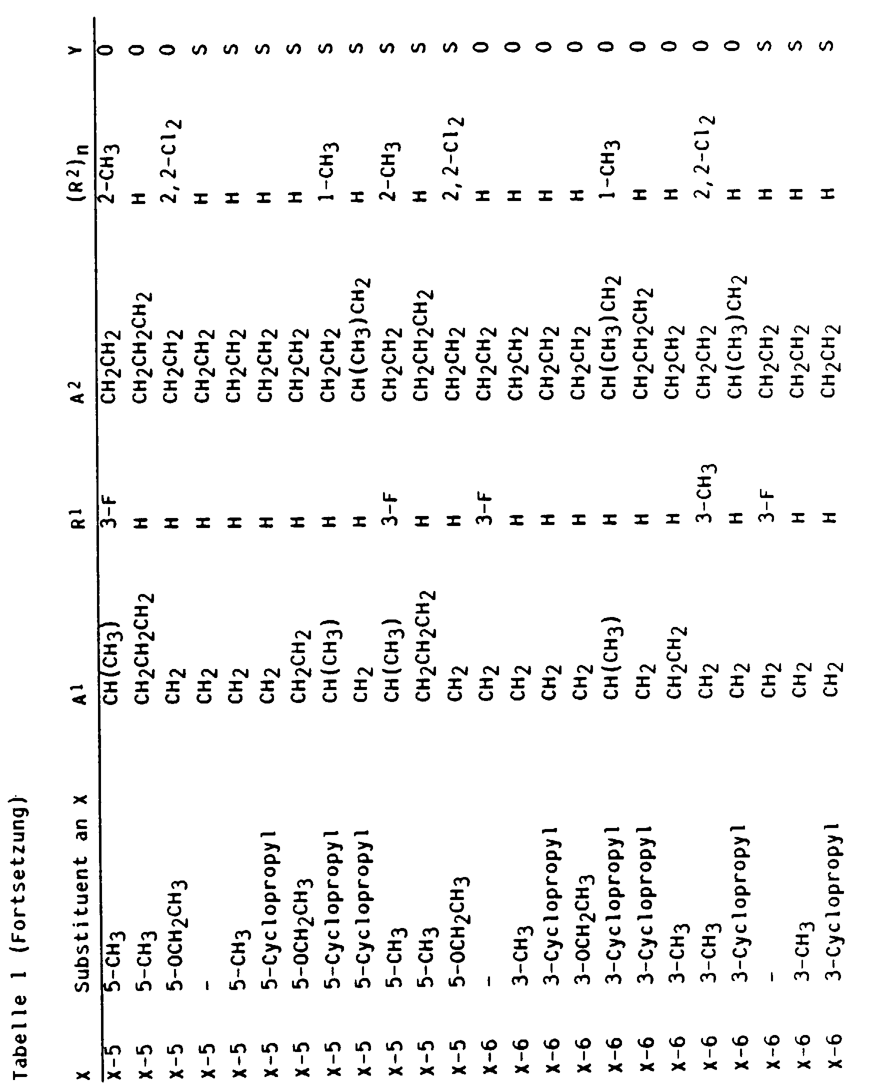

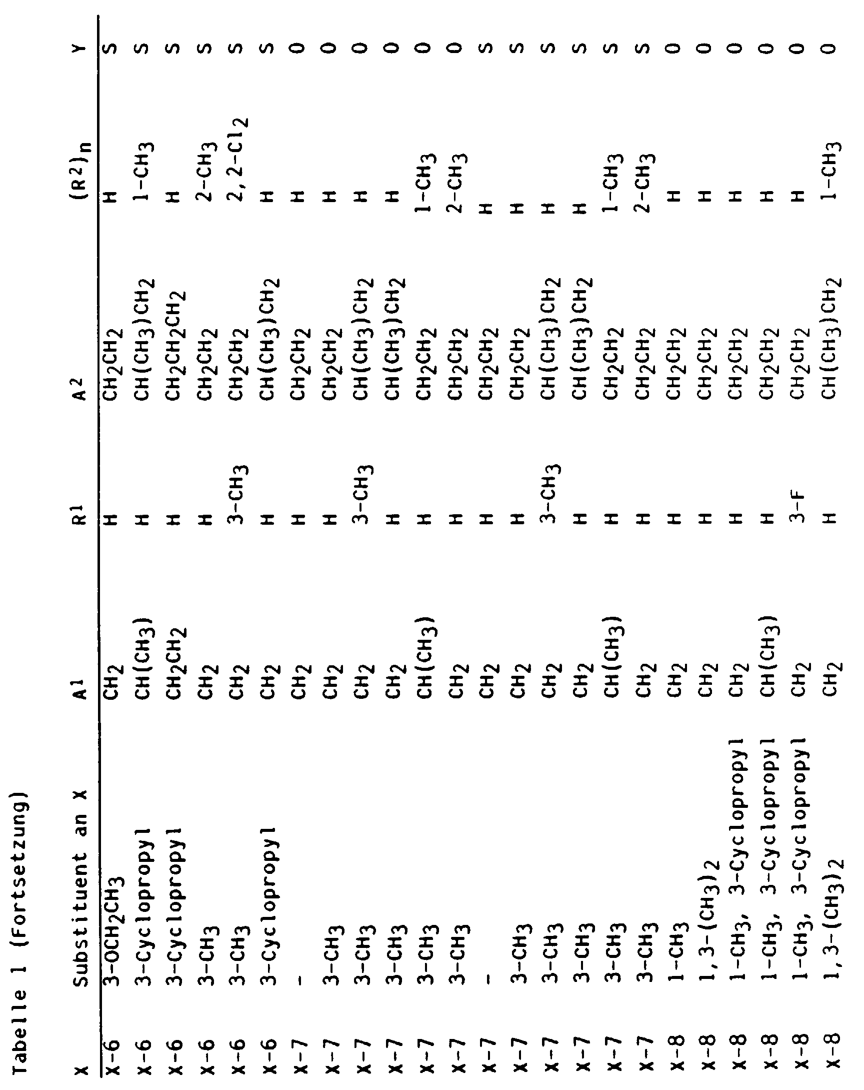

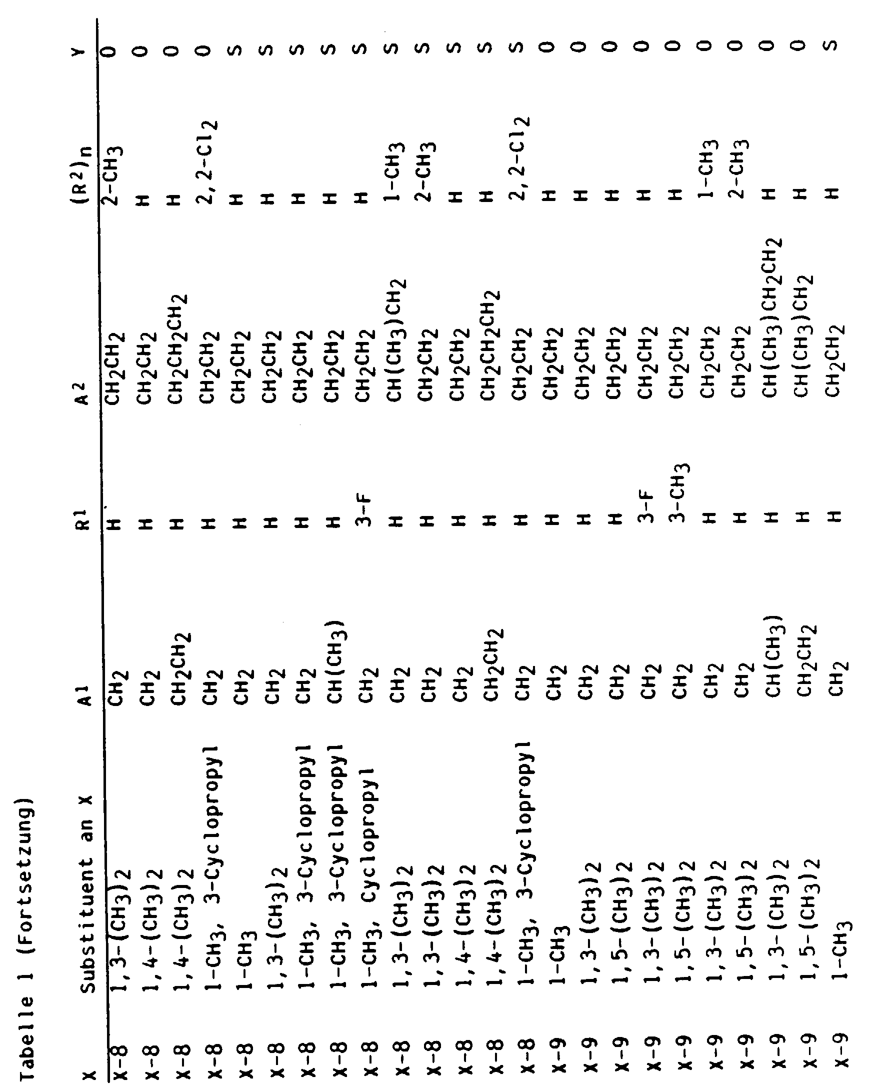

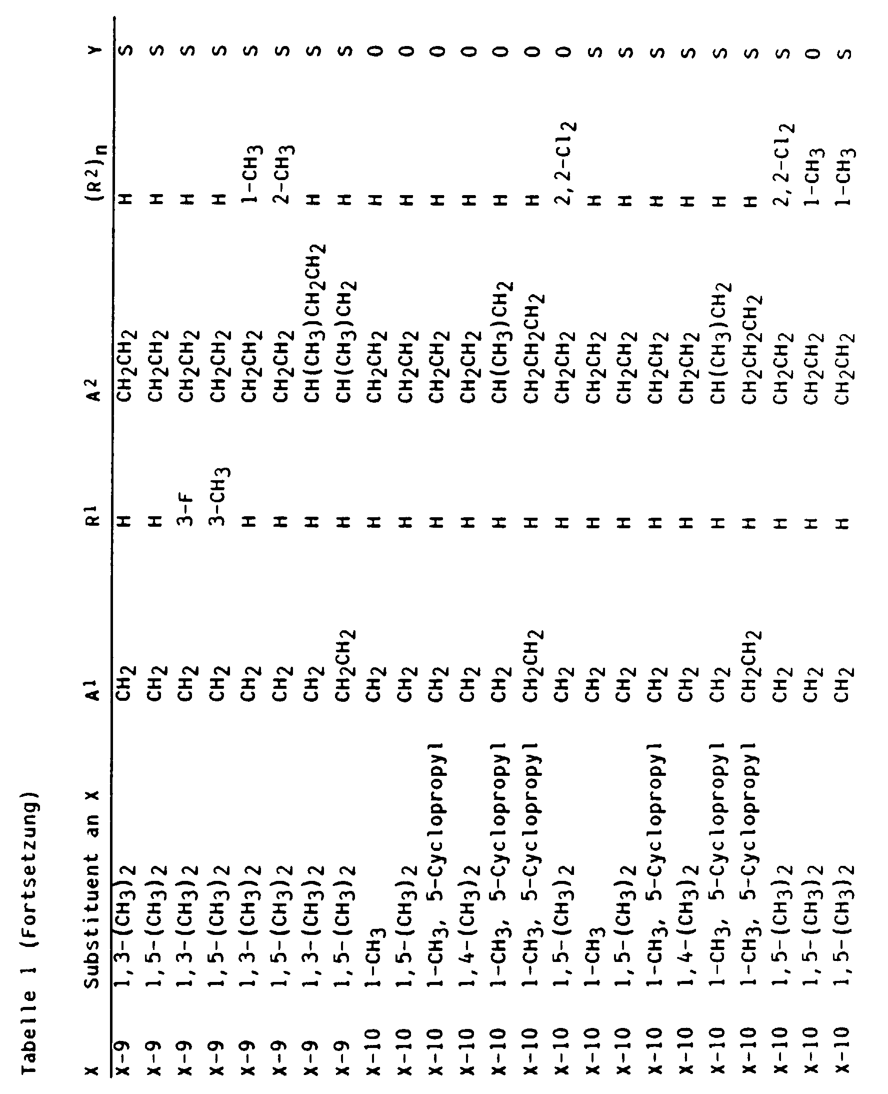

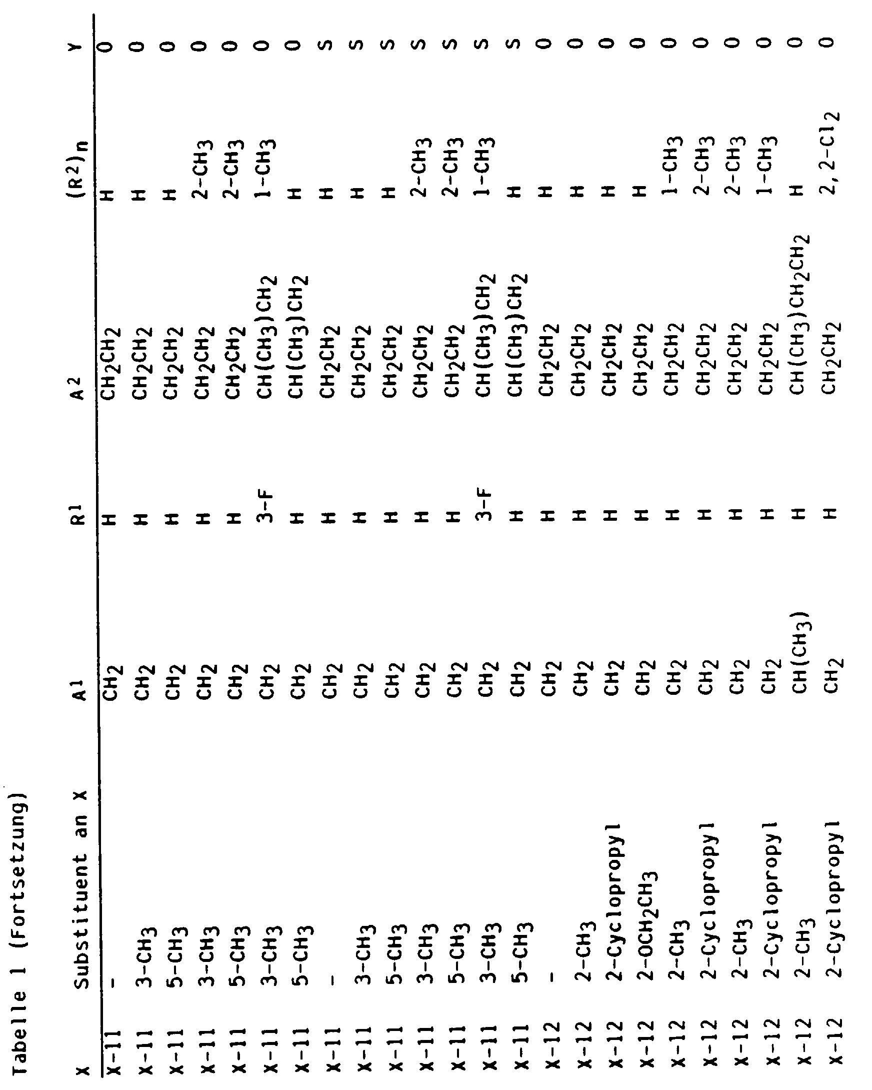

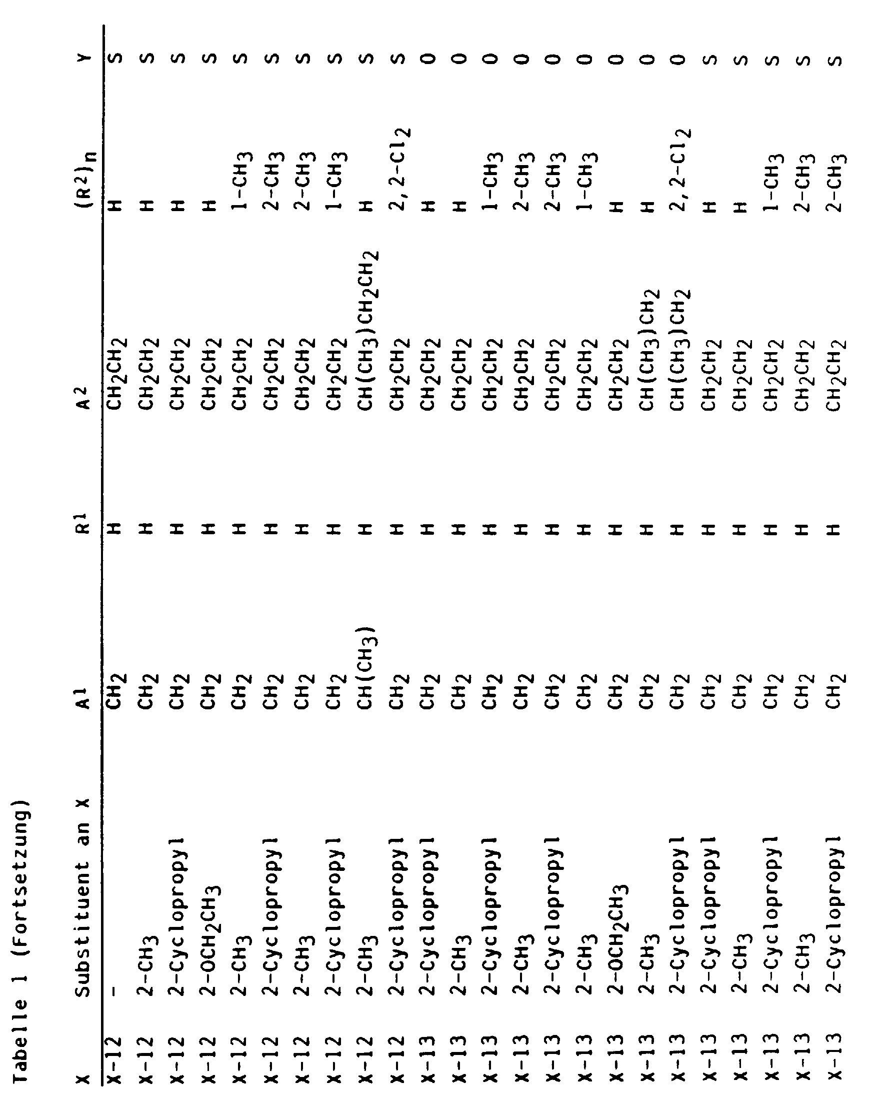

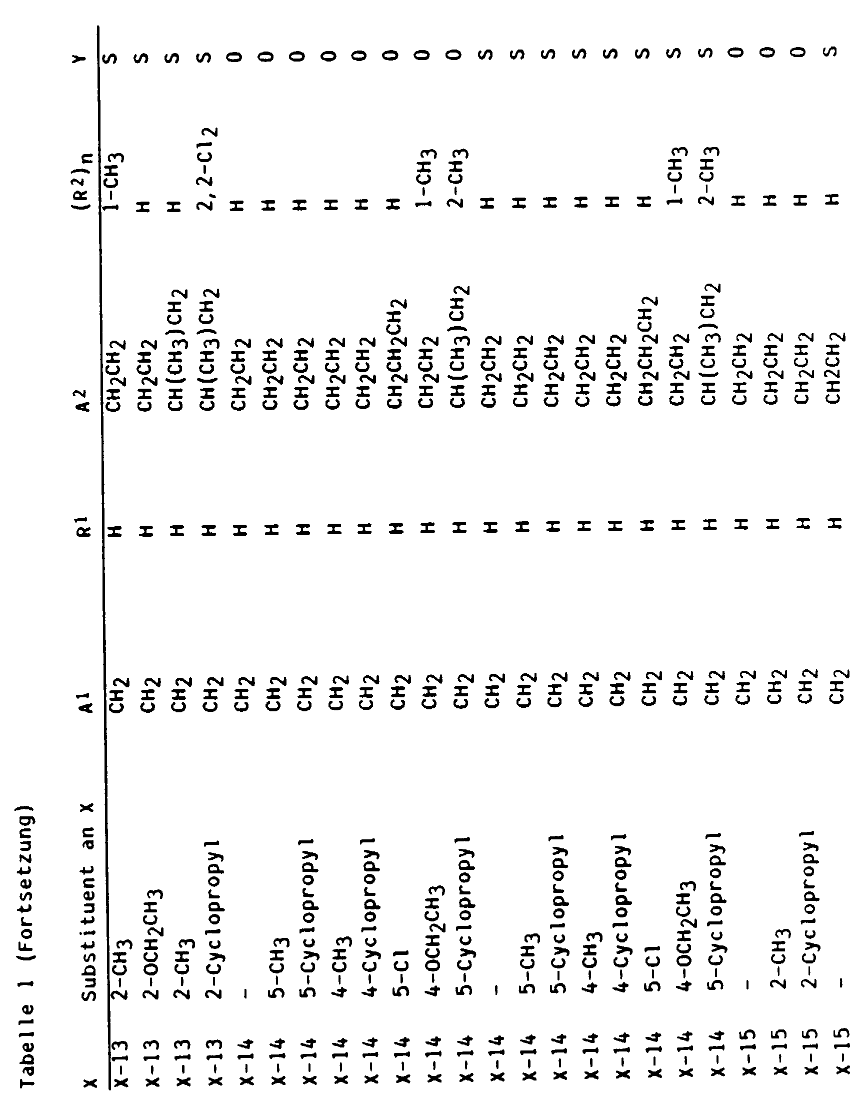

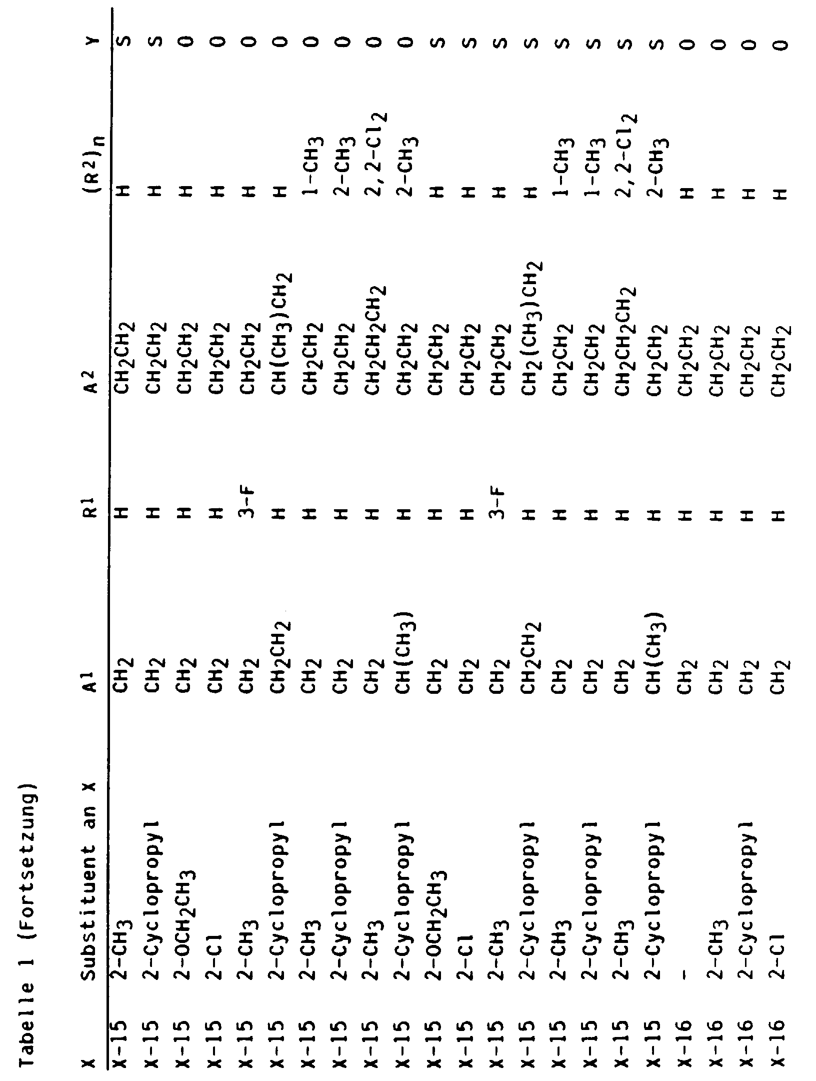

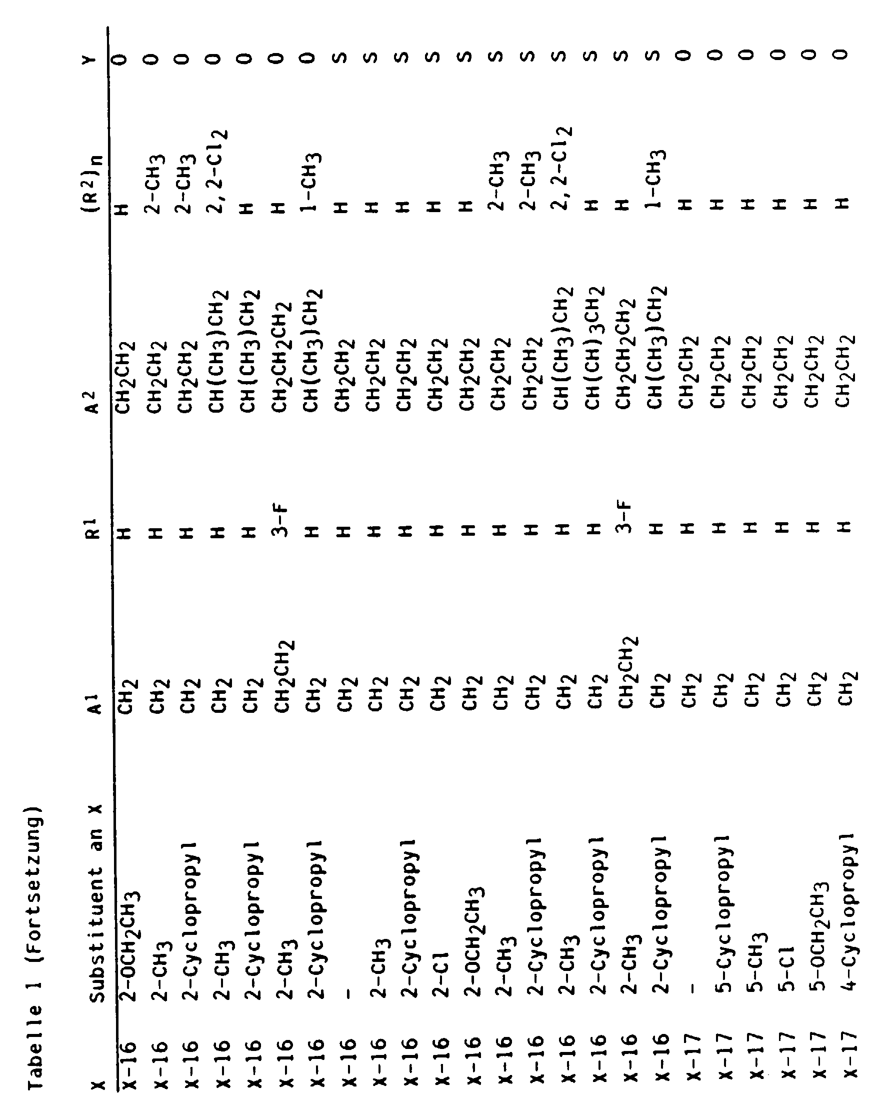

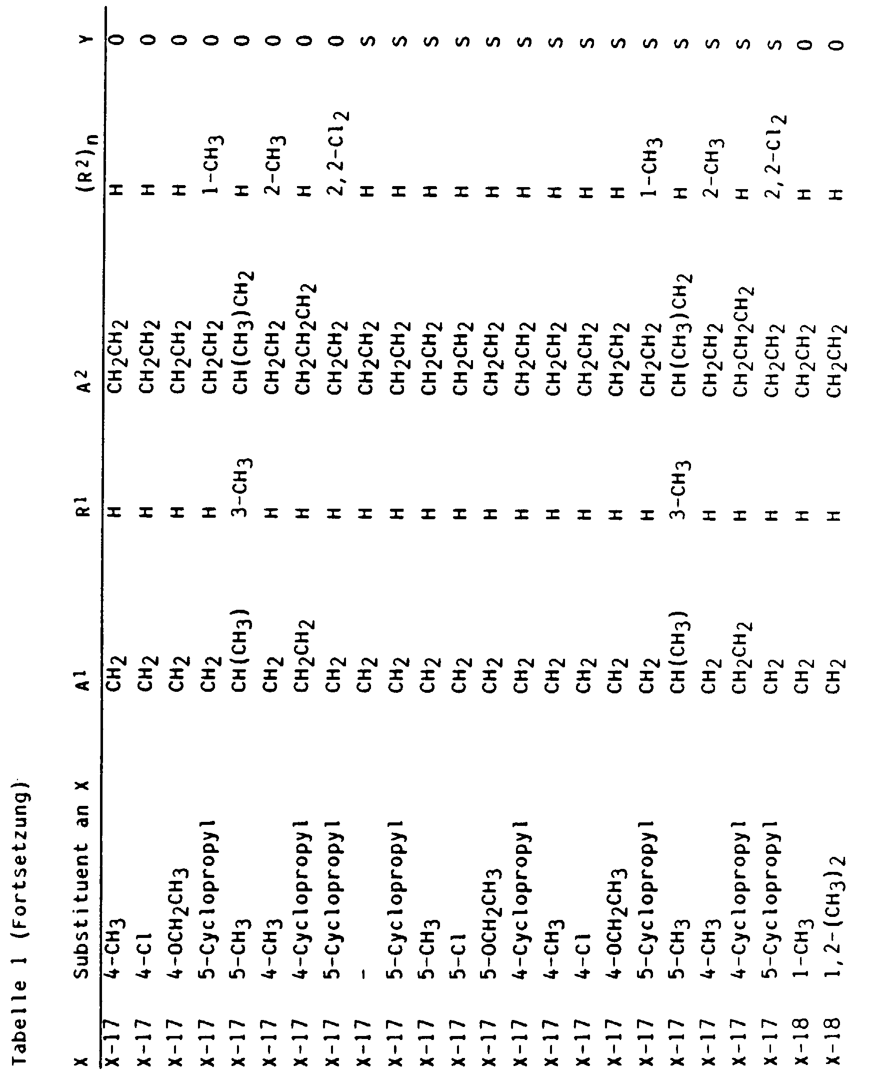

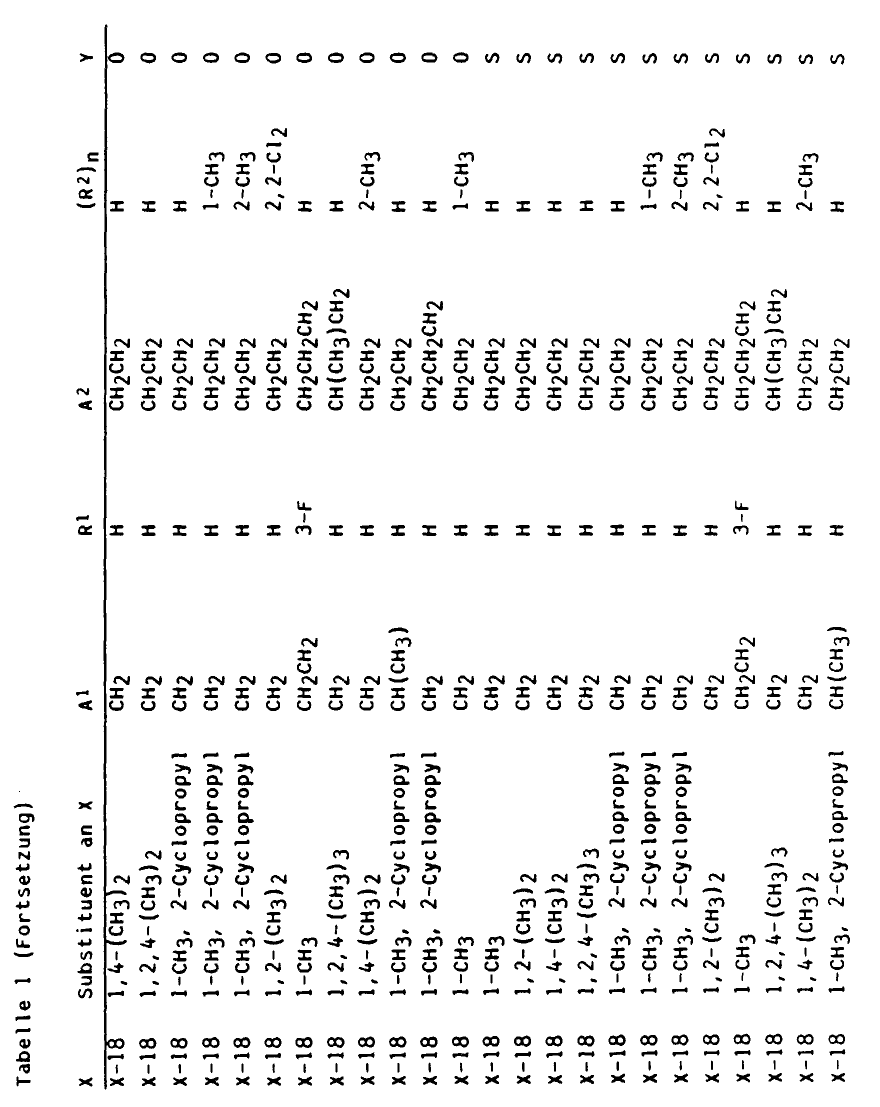

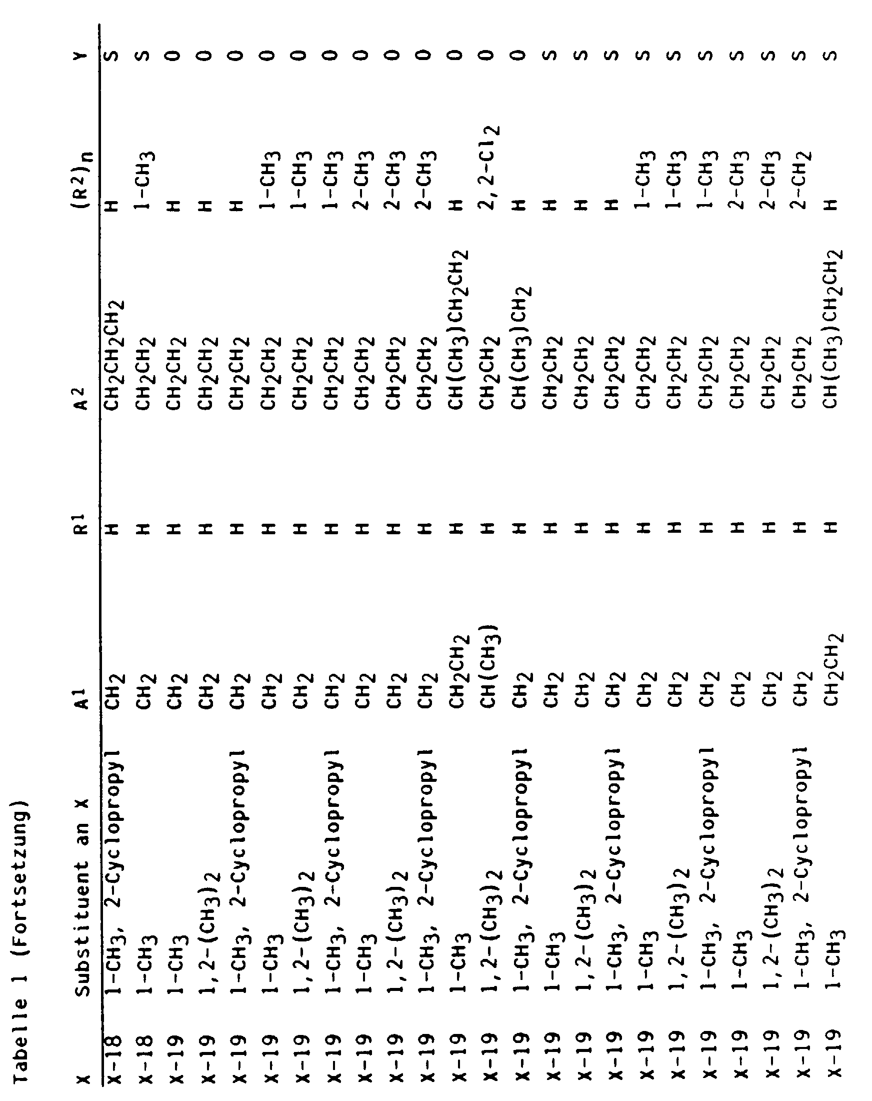

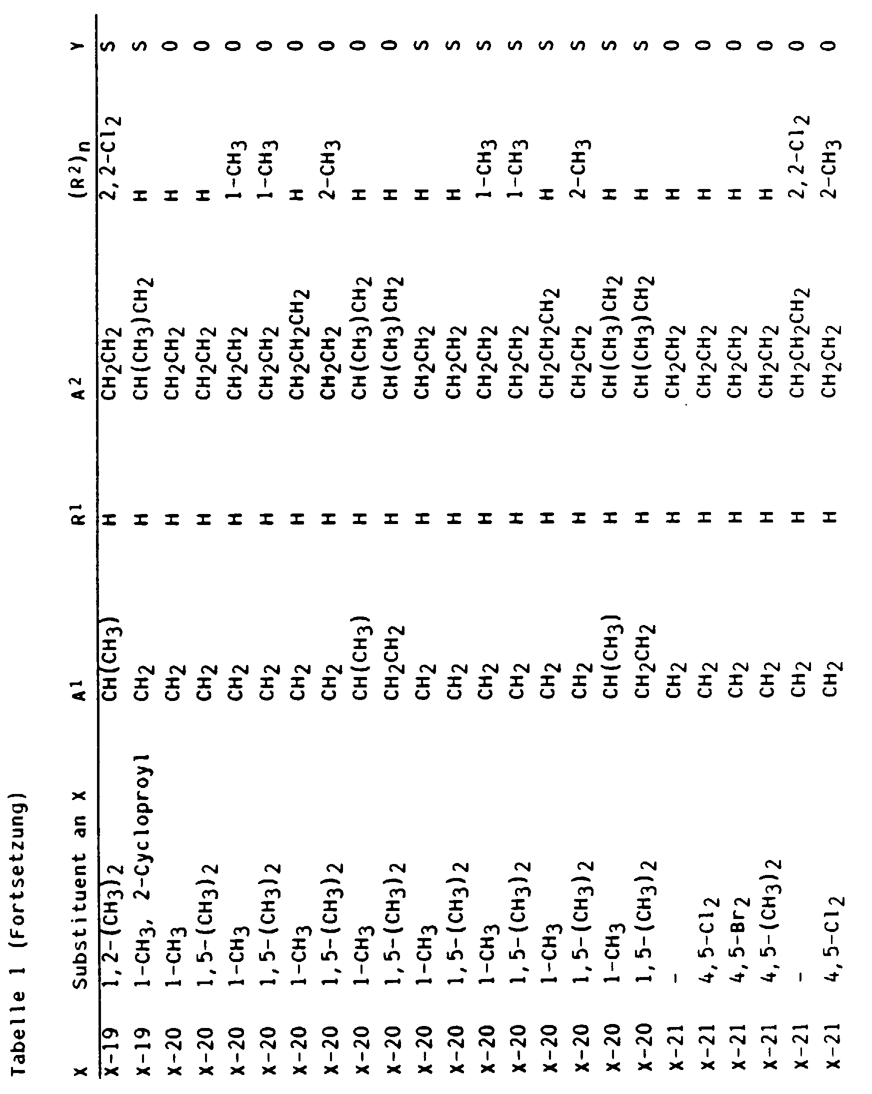

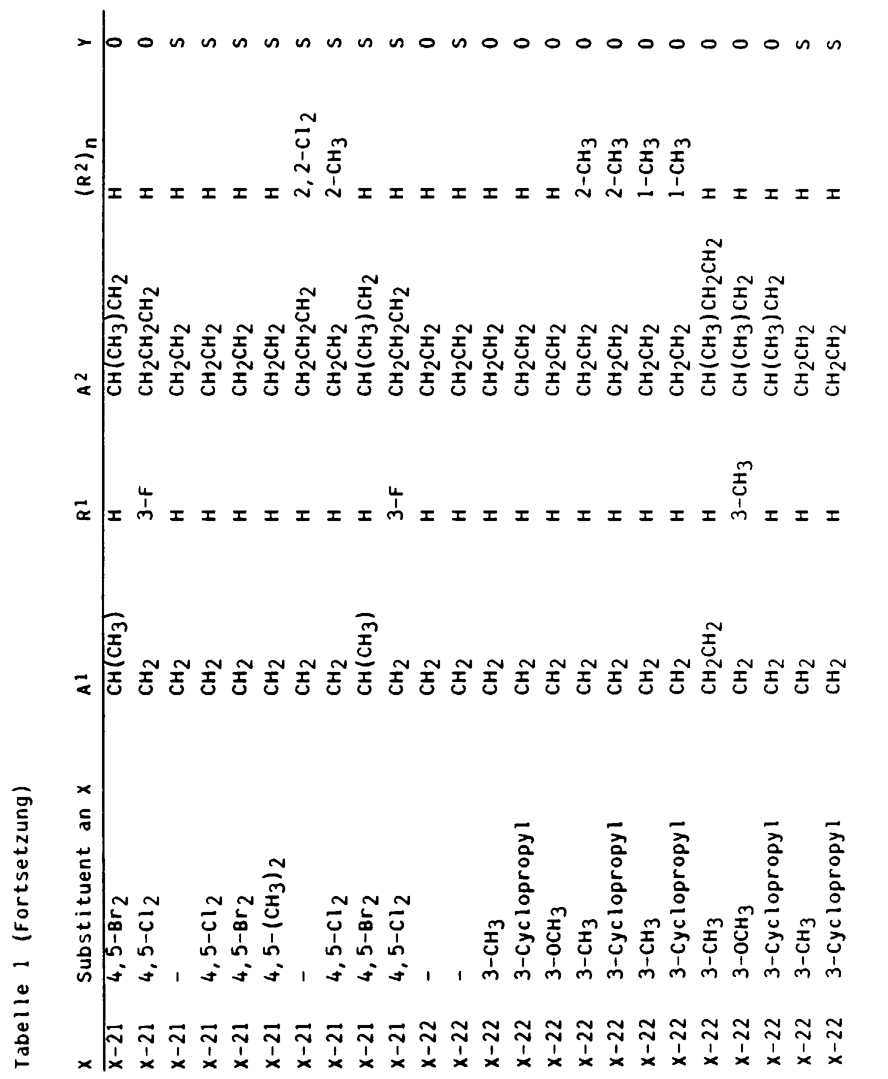

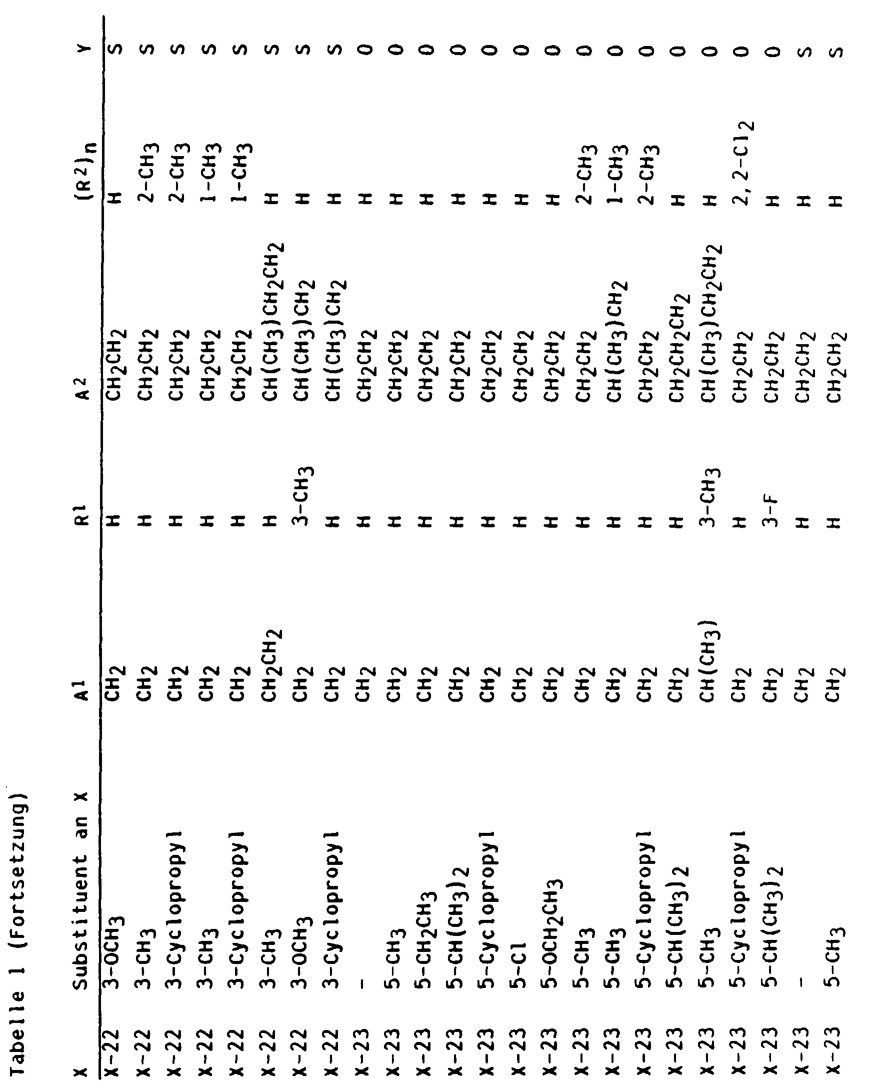

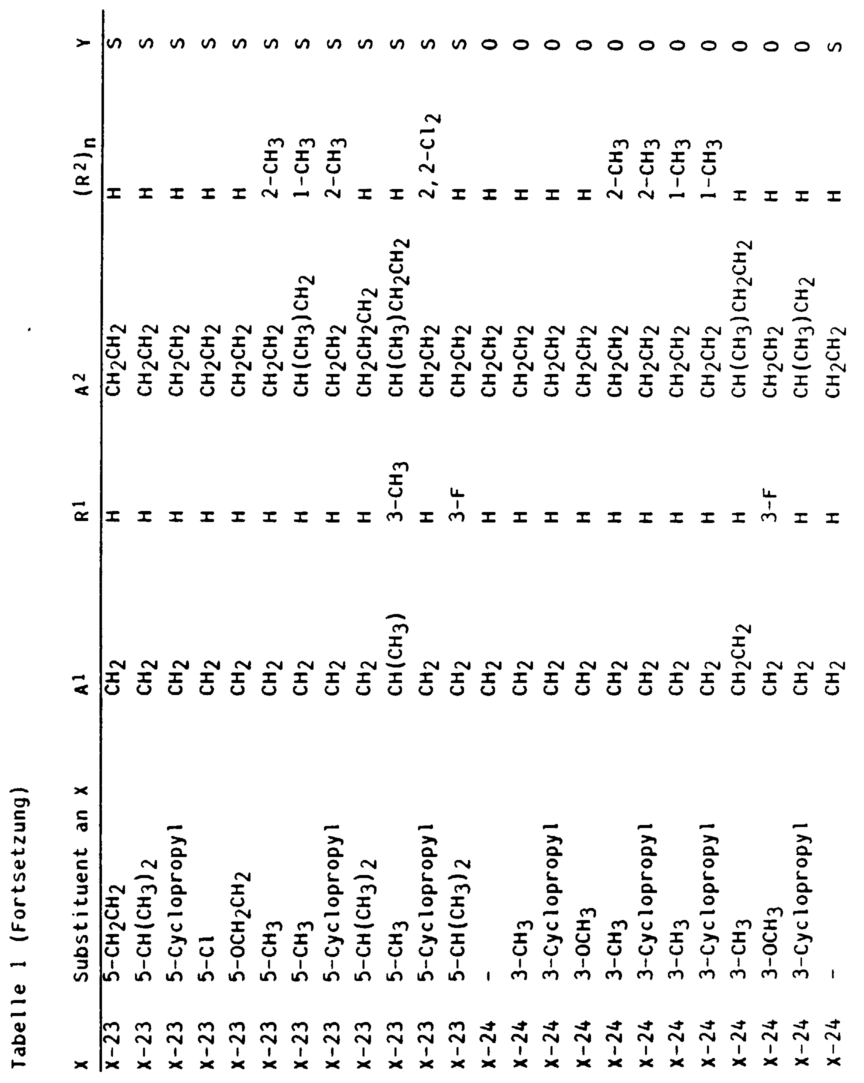

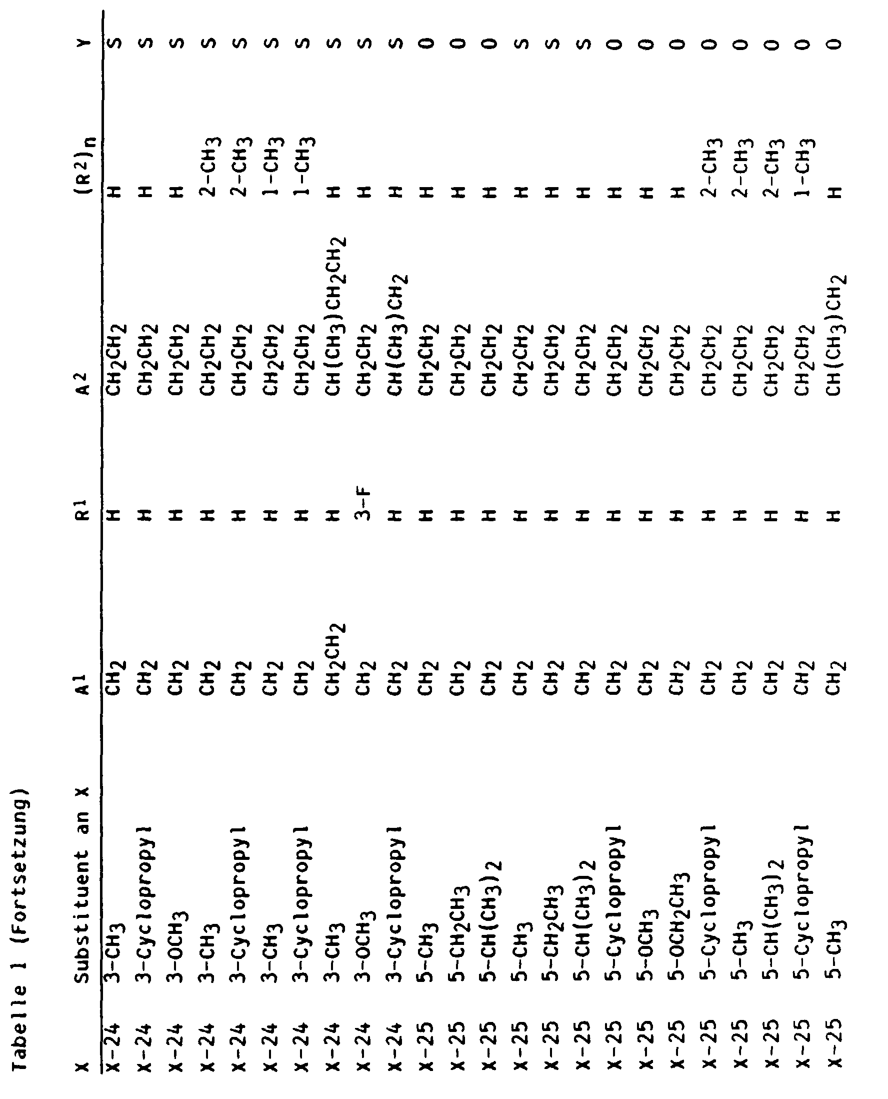

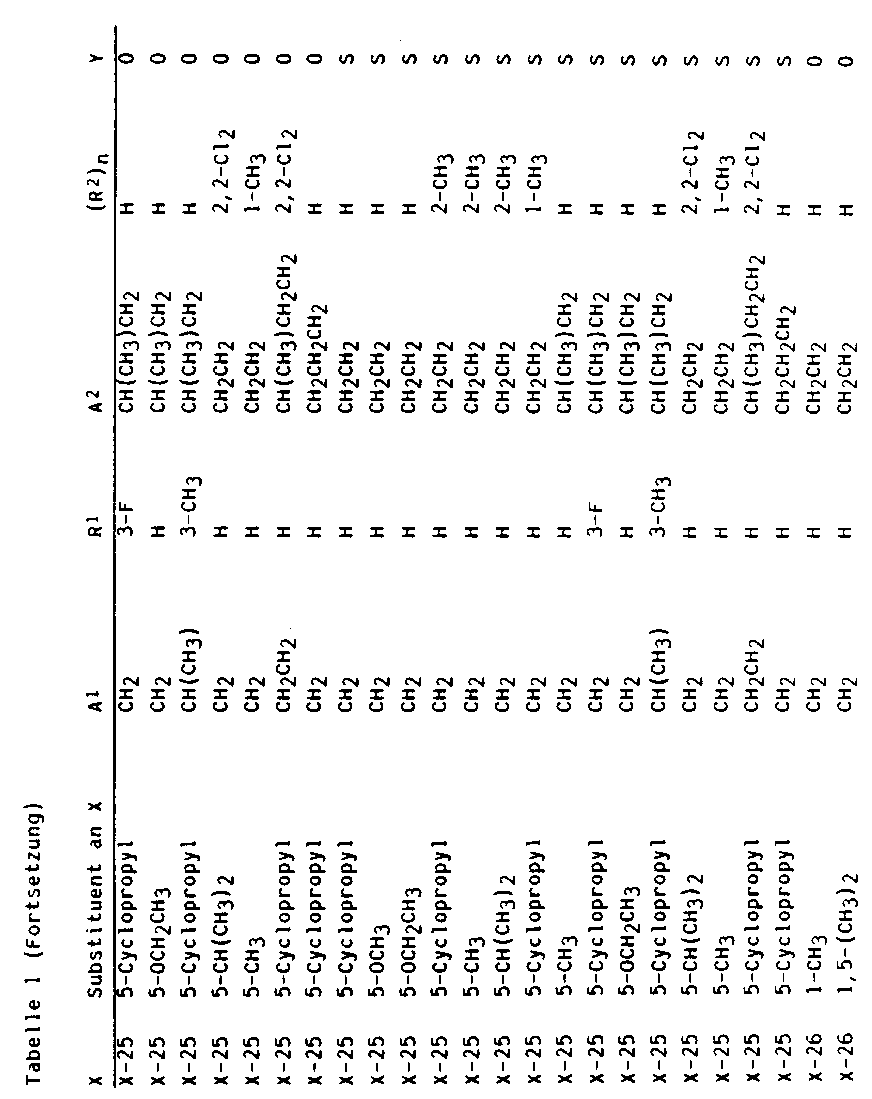

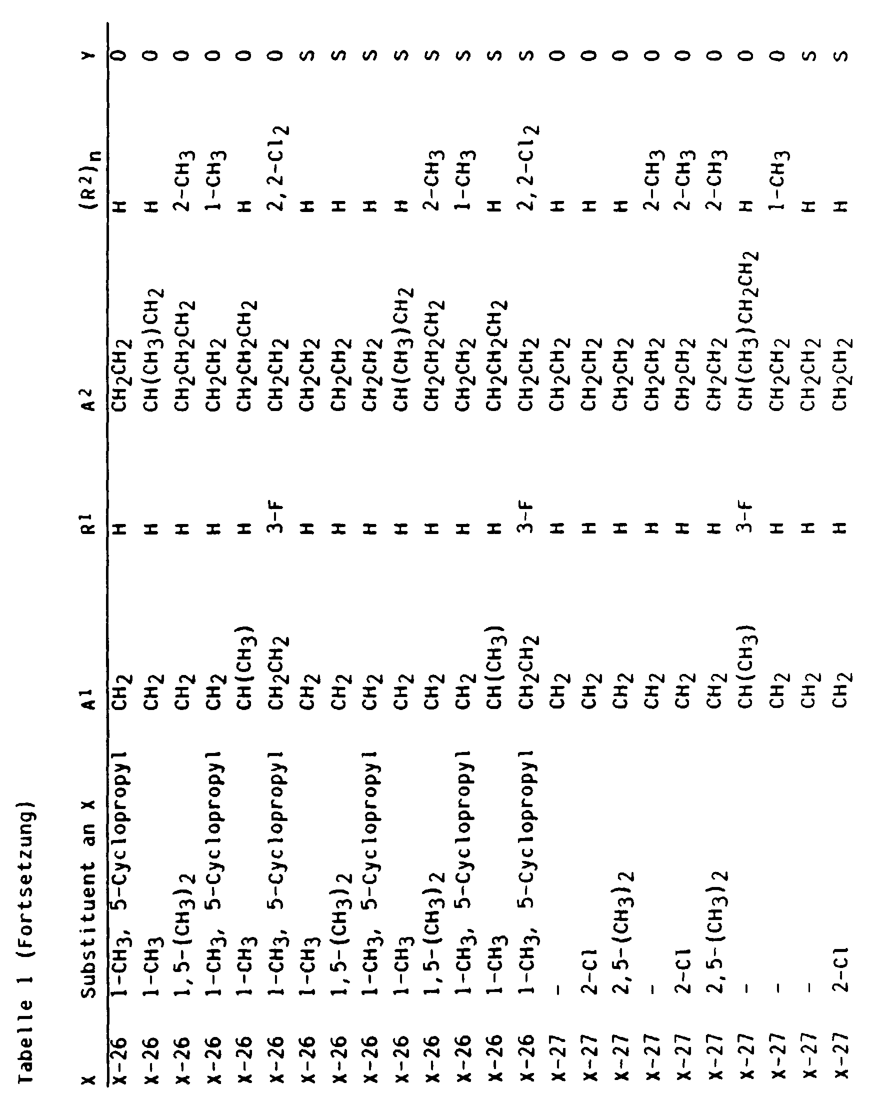

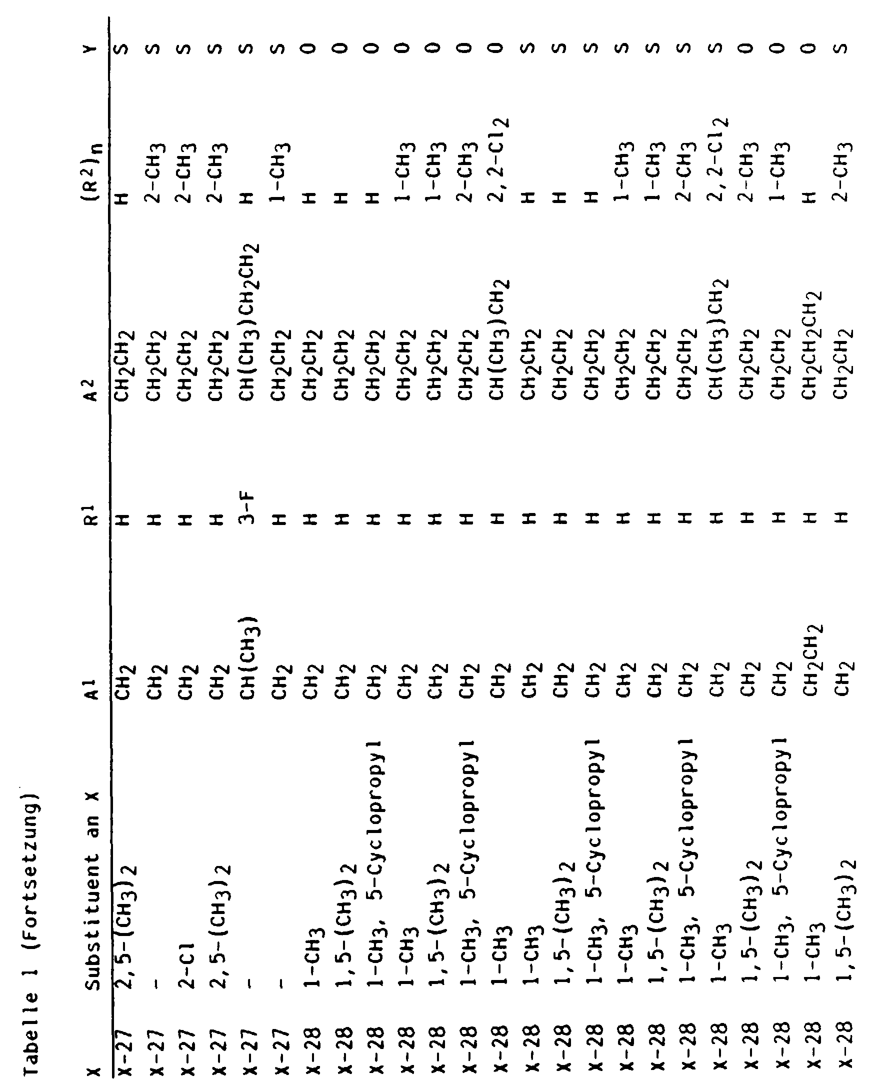

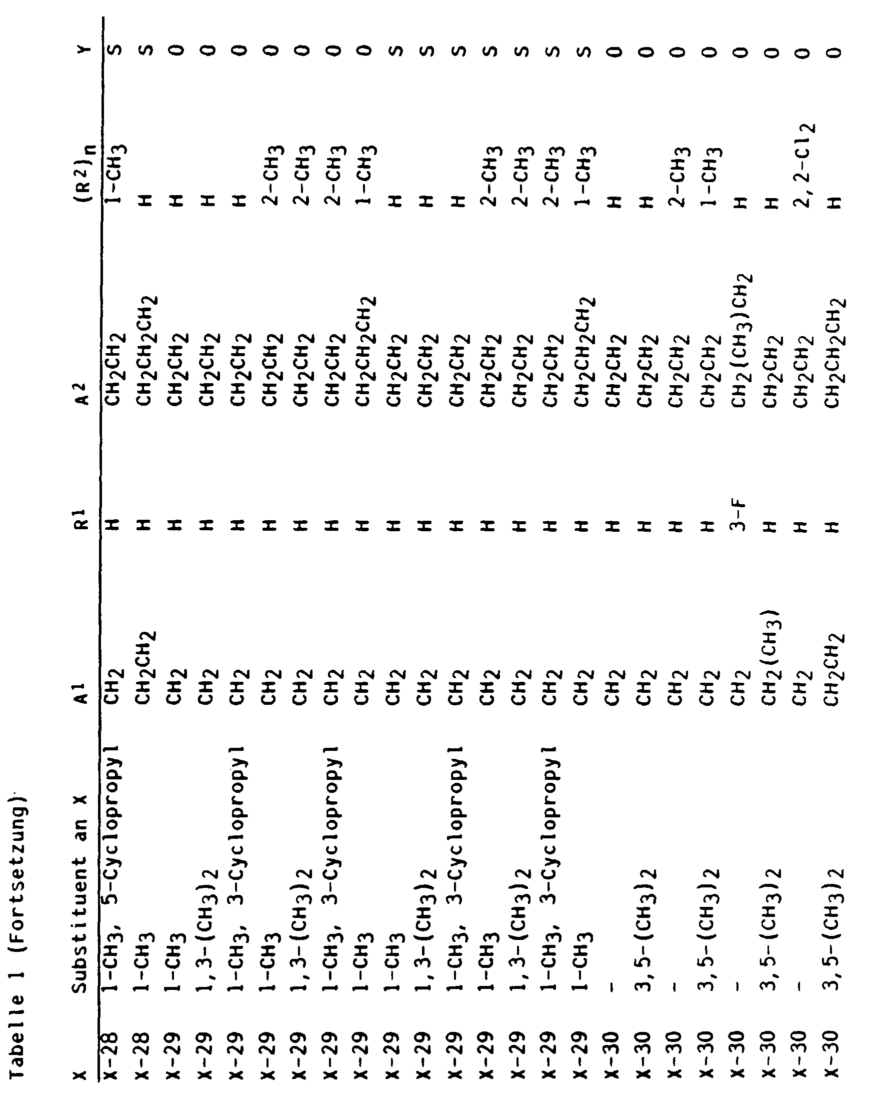

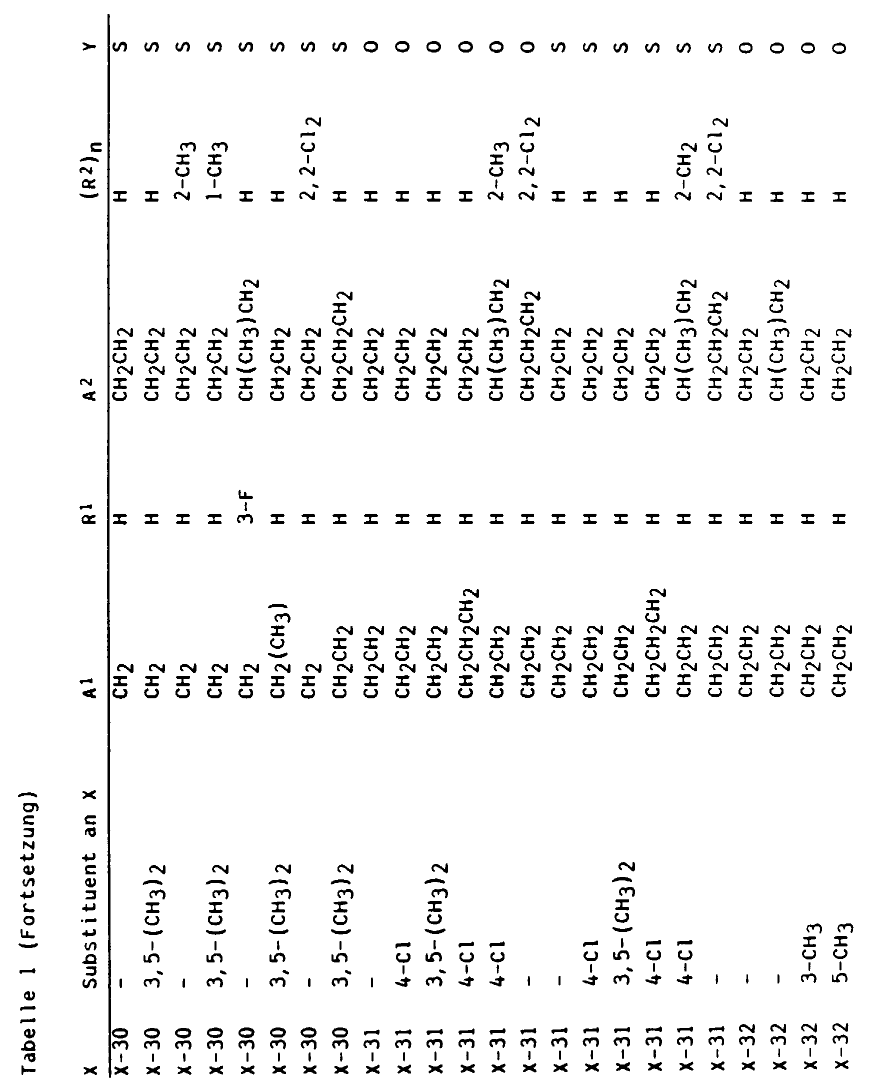

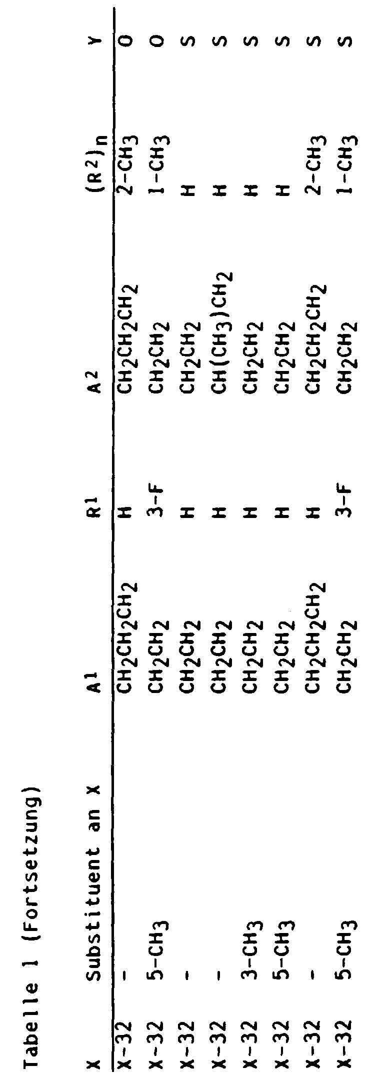

Zur Synthese der Cyclopropancarbonsäureamide und -thioamide I werden als Zwischenprodukte die Phenole der allgemeinen Formel VIII sowie die primären Amine der allgemeinen Formel X verwendet. Die Substituenten R¹, R² und A² in VIII bzw. R¹, A¹, A² und X in X haben dabei die im allgemeinen und im besonderen vorstehend genannte Bedeutung.

Die Verbindungen der Formel I sind geeignet, Schädlinge aus der Klasse der Insekten, Spinnentiere und Nematoden wirksam zu bekämpfen. Sie können im Pflanzenschutz sowie auf dem Hygiene-, Vorratsschutz- und Veterinärsektor als Schädlingsbekämpfungsmittel eingesetzt werden.

Zu den schädlichen Insekten gehören aus der Ordnung der Schmetterlinge (Lepidoptera) beispielsweise Agrotis ypsilon, Agrotis segetum, Alabama argillacea, Anticarsia gemmatalis, Argyresthia conjugella, Autographa gamma, Bupalus piniarius, Cacoecia murinana, Capua reticulana, Cheimatobia brumata, Choristoneura fumiferana, Choristoneura occidentalis, Cirphis unipuncta, Cydia pomonella, Dendrolimus pini, Diaphania nitidalis, Diatraea grandiosella, Earias insulana, Elasmopalpus lignosellus, Eupoecilia ambiguella, Evetria bouliana, Feltia subterranea, Galleria mellonella, Grapholitha funebrana, Grapholitha molesta, Heliothis armigera, Heliothis virescens, Heliothis zea, Hellula undalis, Hibernia defoliaria, Hyphantria cunea, Hyponomeuta malinellus, Keiferia lycopersicella, Lambdina fiscellaria, Laphygma exigua, Leucoptera coffeella, Leucoptera scitella, Lithocolletis blancardella, Lobesia botrana, Loxostege sticticalis, Lymantria dispar, Lymantria monacha, Lyonetia clerkella, Malacosoma neustria, Mamestra brassicae, Orgyia pseudotsugata, Ostrinia nubilalis, Panolis flammea, Pectinophora gossypiella, Peridroma saucia, Phalera bucephala, Phthorimaea operculella, Phyllocnistis citrella, Pieris brassicae, Plathypena scabra, Plutella xylostella, Pseudoplusia includens, Rhyacionia frustrana, Scrobipalpula absoluta, Sitotroga cerealella, Sparganothis pilleriana, Spodoptera frugiperda, Spodoptera littoralis, Spodoptera litura, Thaumatopoea pityocampa, Tortrix viridana, Trichoplusia ni, Zeiraphera canadensis.

Aus der Ordnung der Käfer (Coleoptera) beispielsweise Agrilus sinuatus, Agriotes lineatus, Agriotes obscurus, Amphimallus solstitialis, Anisandrus dispar, Anthonomus grandis, Anthonomus pomorum, Atomaria linearis, Blastophagus piniperda, Blitophaga undata, Bruchus rufimanus, Bruchus pisorum, Bruchus lentis, Byctiscus betulae, Cassida nebulosa, Cerotoma trifurcata, Ceuthorrhynchus assimilis, Ceuthorrynchus napi, Chaetocnema tibialis, Conoderus vespertinus, Crioceris asparagi, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Oulema oryzae, Otiorrhynchus sulcatus, Otiorrhynchus ovatus, Phaedon cochleariae, Phyllotreta chrysocephala, Phyllophaga sp., Phyllopertha horticola, Phyllotreta nemorum, Phyllotreta striolata, Popillia japonica, Sitona lineatus, Sitophilus granaria.

Aus der Ordnung der Zweiflügler (Diptera) beispielsweise Aedes aegypti, Aedes vexans, Anastrepha ludens, Anopheles maculipennis, Ceratitis capitata, Chrysomya bezziana, Chrysomya hominivorax, Chrysomya macellaria, Contarinia sorghicola, Cordylobia anthropophaga, Culex pipiens, Dacus cucurbitae, Dacus oleae, Dasineura brassicae, Fannia canicularis, Gasterophilus intestinalis, Glossina morsitans, Haematobia irritans, Haplodiplosis equestris, Hylemyia platura, Hypoderma lineata, Liriomyza sativae, Liriomyza trifolii, Lucilia caprina, Lucilia cuprina, Lucilia sericata, Lycoria pectoralis, Mayetiola destructor, Musca domestica, Muscina stabulans, Oestrus ovis, Oscinella frit, Pegomya hyoscyami, Phorbia antiqua, Phorbia brassicae, Phorbia coarctata, Rhagoletis cerasi, Rhagoletis pomonella, Tabanus bovinus, Tipula oleracea, Tipula paludosa.

Aus der Ordnung der Thripse (Thysanoptera) beispielsweise Frankliniella fusca, Frankliniella occidentalis, Frankliniella tritici, Scirtothrips citri, Thrips oryzae, Thrips palmi, Thrips tabaci.

Aus der Ordnung der Hautflügler (Hymenoptera) beispielsweise Athalia rosae, Atta cephalotes, Atta sexdens, Atta texana, Hoplocampa minuta, Hoplocampa testudinea, Monomorium pharaonis, Solenopsis geminata, Solenopsis invicta.

Aus der Ordnung der Wanzen (Heteroptera) beispielsweise Acrosternum hilare, Blissus leucopterus, Cyrtopeltis notatus, Dysdercus cingulatus, Dysdercus intermedius, Eurygaster integriceps, Euschistus impictiventris, Leptoglossus phyllopus, Lygus lineolaris, Lygus pratensis, Nezara 5 viridula, Piesma quadrata, Solubea insularis, Thyanta perditor.

Aus der Ordnung der Pflanzensauger (Homoptera) beispielsweise Acyrthosiphon onobrychis, Adelges laricis, Aphidula nasturtii, Aphis fabae, Aphis pomi, Aphis sambuci, Brachycaudus cardui, Brevicoryne brassicae, Cerosipha gossypii, Dreyfusia nordmannianae, Dreyfusia piceae, Dysaphis radicola, Dysaulacorthum pseudosolani, Empoasca fabae, Macrosiphum avenae, Macrosiphum euphorbiae, Macrosiphon rosae, Megoura viciae, Metopolophium dirhodum, Myzodes persicae, Myzus cerasi, Nilaparvata lugens, Pemphigus bursarius, Perkinsiella saccharicida, Phorodon humuli, Psylla mali, Psylla piri, Rhopalomyzus ascalonicus, Rhopalosiphum maidis, Sappaphis mala, Sappaphis mali, Schizaphis graminum, Schizoneura lanuginosa, Trialeurodes vaporariorum, Viteus vitifolii.

Aus der Ordnung der Termiten (Isoptera) beispielsweise Calotermes flavicollis, Leucotermes flavipes, Reticulitermes lucifugus, Termes natalensis.

Aus der Ordnung der Geradflügler (Orthoptera) beispielsweise Acheta domestica, Blatta orientalis, Blattella germanica, Forficula auricularia, Gryllotalpa gryllotalpa, Locusta migratoria, Melanoplus bivittatus, Melanoplus femur-rubrum, Melanoplus mexicanus, Melanoplus sanguinipes` Melanoplus spretus, Nomadacris septemfasciata, Periplaneta americana, Schistocerca americana, Schistocerca peregrina, Stauronotus maroccanus, Tachycines asynamorus.

Aus der Klasse der Arachnoidea beispielsweise Spinnentiere (Acarina) wie Amblyomma americanum, Amblyomma variegatum, Argas persicus, Boophilus annulatus, Boophilus decoloratus, Boophilus microplus, Brevipalpus phoenicis, Bryobia praetiosa, Dermacentor silvarum, Eotetranychus carpini, Eriophyes sheldoni, Hyalomma truncatum, Ixodes ricinus, Ixodes rubicundus, Ornithodorus moubata, Otobius megnini, Paratetranychus pilosus, Dermanyssus gallinae, Phyllocaptruta oleivora, Polyphagotarsonemus latus, Psoroptes ovis, Rhipicephalus appendiculatus, Rhipicephalus evertsi, Sarcoptes scabiei, Tetranychus cinnabarinus, Tetranychus kanzawai, Tetranychus pacificus, Tetranychus telarius, Tetranychus urticae.

Aus der Klasse der Nematoden beispielsweise Wurzelgallennematoden, z.B. Meloidogyne hapla, Meloidogyne incognita, Meloidogyne javanica, Zysten bildende Nematoden` z.B. Globodera rostochiensis, Heterodera avenae, Heterodera glycinae, Heterodera schachtii, Heterodera trifolii, Stock- und Blattälchen, z.B. Belonolaimus longicaudatus, Ditylenchus destructor, Ditylenchus dipsaci, Heliocotylenchus multicinctus, Longidorus elongatus, Radopholus similis, Rotylenchus robustus, Trichodorus primitivus, Tylenchorhynchus claytoni, Tylenchorhynchus dubius, Pratylenchus neglectus, Pratylenchus penetrans, Pratylenchus curvitatus, Pratylenchus goodeyi.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulver, öldispersionen) durch Zusatz von Wasser bereitet werden. Zur Herstellung von Emulsionen, Pasten oder öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate und Fettsäuren sowie deren Alkali- und Erdalkalisalze, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Ligninsulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Die Formulierungen enthalten im allgemeinen zwischen 0,01 und 95 Gew.%, vorzugsweise zwischen 0,1 und 90 Gew.% des Wirkstoffs. Die Wirkstoffe werden dabei in einer Reinheit von 90 % bis 100 %, vorzugsweise 95 % bis 100 % (nach NMR-Spektrum) eingesetzt.

Beispiele für Formulierungen sind:
- I.: 5 Gew.-Teile der Verbindung Nr. 2.06 werden mit 95 Gew.-Teilen feinteiligem Kaolin innig vermischt. Man erhält auf diese Weise ein Stäubemittel, das 5 Gew.% des Wirkstoffs enthält.
- II.: 30 Gew.-Teile der Verbindung Nr. 2.11 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit (Wirkstoffgehalt 23 Gew.%).
- III.: 10 Gew.-Teile der Verbindung Nr. 2.08 werden in einer Mischung gelöst, die aus 90 Gew.-Teilen Xylol, 6 Gew.-Teilen des Anlagerungsproduktes von 8 bis 10 Mol Ethylenoxid an 1 Mol ölsäure-N-monoethanolamid, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht (Wirkstoffgehalt 9 Gew.%).
- IV.: 20 Gew.-Teile der Verbindung Nr. 2.14 werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5 Gew.-Teilen des Anlagerungsproduktes von 40 Mol Ethylenoxid an 1 Mol Ricinusöl besteht (Wirkstoffgehalt 16 Gew.%).
- V.: 80 Gew.-Teile der Verbindung Nr. 2.03 werden mit 3 Gew.-Teilen des Natriumsalzes der Diisobutylnaphthalin-alpha-sulfonsäure, 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle vermahlen (Wirkstoffgehalt 80 Gew.%).
- VI.: Man vermischt 90 Gew.-Teile der Verbindung Nr. 2.10 mit 10 Gew.-Teilen N-Methyl-a-pyrrolidon und erhält eine Lösung, die zur Anwendung in Form kleinster Tropfen geeignet ist (Wirkstoffgehalt 90 Gew.%).
- VII.: 20 Gew.-Teile der Verbindung Nr. 2.04 werden in einer Mischung gelöst, die aus 40 Gew.-Teilen Cyclohexanon, 30 Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl besteht. Durch Eingießen und feines Verteilen der Lösung in 100 000 Gew.-Teilen Wasser erhält man eine wäßrige Dispersion, die 0,02 Gew.% des Wirkstoffs enthält.
- VIII.: 20 Gew.-Teile des Wirkstoffs Nr. 2.15 werden mit 3 Gew.-Teilen des Natriumsalzes der Diisobutylnaphthalin-α-sulfonsäure, 17 Gew.-Teilen des Natriumsalzes einer Ligninsulfonsäure aus einer Sulfit-Ablauge und 60 Gew.-Teilen pulverförmigem Kieselsäuregel gut vermischt und in einer Hammermühle vermahlen. Durch feines Verteilen der Mischung in 20 000 Gew.-Teilen Wasser erhält man eine Spritzbrühe, die 0,1 Gew.% des Wirkstoffs enthält.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Wirkstoffkonzentrationen in den anwendungsfertigen Zubereitungen können in größeren Bereichen variiert werden.

Im allgemeinen liegen sie zwischen 0,0001 und 10 %, vorzugsweise zwischen 0,01 und 1 %.

Die Wirkstoffe können auch mit gutem Erfolg im Ultra-Low-volume-Verfahren (ULV) verwendet werden, wobei es möglich ist, Formulierungen mit mehr als 95 Gew.% Wirkstoff oder sogar den Wirkstoff ohne Zusätze auszubringen.

Die Aufwandmenge an Wirkstoff beträgt unter Freilandbedingungen 0,01 bis 10 kg/ha, vorzugsweise 0,1 bis 1 kg/ha.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

### Herstellungsbeispiele

Die nachstehend wiedergegebenen Vorschriften zur Herstellung der Verbindungen wurden unter Abwandlung der Edukte zur Herstellung weiterer erfindungsgemäßer Verbindungen genutzt. Die entsprechenden Verbindungen und ihre physikalischen Daten sind den anschließenden Tabellen zu entnehmen.

A: Herstellung der Zwischenprodukte der allgemeinen Formel VIII 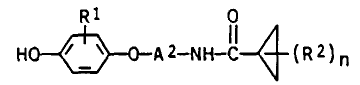 Cyclopropancarbonsäure-N-2-(4-Hydroxyphenoxy-)ethylamid (R¹=R²=H; A²=CH₂CH₂)

Eine Lösung von 63,7 g (0,23 mol) Cyclopropancarbonsäure-N-2-(4-tert.-butoxy-phenoxy-)ethylamid in 1000 ml Ethanol/Wasser (10/1) wurde mit 20 ml (∼1 eq) konz. HCl versetzt und 2 h unter Rückfluß erhitzt. Das Lösungsmittel wurde eingeengt, der Rückstand mit Wasser versetzt, die Mischung auf pH 7 eingestellt und der ausgefallene Niederschlag abgesaugt. Ausbeute: 46,4 g (91 %) weiße Kristalle, Fp 115°C.

Herstellung der Wirkstoffe der allgemeinen Formel I

### Beispiel 1

### 5-(4-Cyclopropylcarbonylaminoethoxyphenoxy-)methyl-3-isopropyl-isoxazol (Verb. Nr. 2.02 in Tabelle 2)

Eine Suspension von 1,3 g (1 eq) NaH (80 %ige Dispersion in Mineralöl) in 60 ml DMF wurde bei RT tropfenweise mit einer Lösung von 9,7 g (44 mmol) der bei A hergestellten Verbindung in 70 ml DMF versetzt und 1 h auf 80°C erwärmt. Nach Zugabe einer Lösung von 7,0 g (44 mmol) 5-Chlormethyl-3-isopropyl-isoxazol in 50 ml DMF wurde 6 h auf 120°C erhitzt, anschließend das Lösungsmittel im Vakuum abgezogen, der Rückstand in Ethylacetat aufgenommen und mit Wasser gewaschen. Einengen des Lösungsmittels und Umkristallisation des erhaltenen Feststoffs aus Ethanol ergab 8,7 g (57 %) des gewünschten Phenolethers, Fp: 76 - 80°C.

### Beispiel 2

### 5-(4-Cyclopropylcarbonylaminoethoxyphenoxy-)methyl-2-cyclopropyl-1,3,4-thiadiazol (Verb. Nr. 2.05 in Tabelle 2)

Eine Umsetzung von 9,7 g (44 mmol) VII a und 7,6 g (44 mmol) 5-Chlormethyl-2-cyclopropyl-1,3,4-thiadiazol analog Synthesebeispiel 2 ergab nach Umkristallisation des Rohprodukts aus Ethanol 7,9 g (50 %) des gewünschten Phenolethers, Fp: 105 - 112°C.

### Beispiel 3

### 5-(4-Cyclopropylthiocarbonylaminoethoxyphenoxy-)methyl-3-isopropylisoxazol (Verb. Nr. 2.10 in Tabelle 2)

Eine Suspension von 5,85 g (17 mmol) der Verbindung 2.02 und 6,9 g (17 mmol) Lawesson's Reagenz in 100 ml Toluol wurde 1 h auf 80°C erwärmt. Das Lösungsmittel wurde entfernt und das Rohprodukt chromatographisch vorgereinigt (Kieselgel, Cyclohexan/Ethylacetat = 1/1). Umkristallisation des Produkts aus Cyclohexan/Ethylacetat ergab 2,7 g (44 %) des gewünschten Thioamids, Fp: 95 - 102°C.

### Beispiel 4

### 5-(4-Cyclopropylthiocarbonylaminoethoxyphenoxy-)methyl-2-cyclopropyl-1,3,4-thiadiazol (Verb. Nr. 2.13 in Tabelle 2)

Eine Suspension von 4,3 g (12 mmol) der Verbindung 2.05 und 5,6 g (13,8 mmol) Lawesson's Reagenz in 100 ml Toluol wurde 1,5 h auf 80°C erwärmt. Nach Entfernung des Lösungsmittels und chromatographischer Reinigung des Rohprodukts (Kieselgel, Cyclohexan/Ethylacetat = 1/1) wurden 3,9 g (87 %) des gewünschten Thioamids erhalten, Fp: 114 - 116°C.

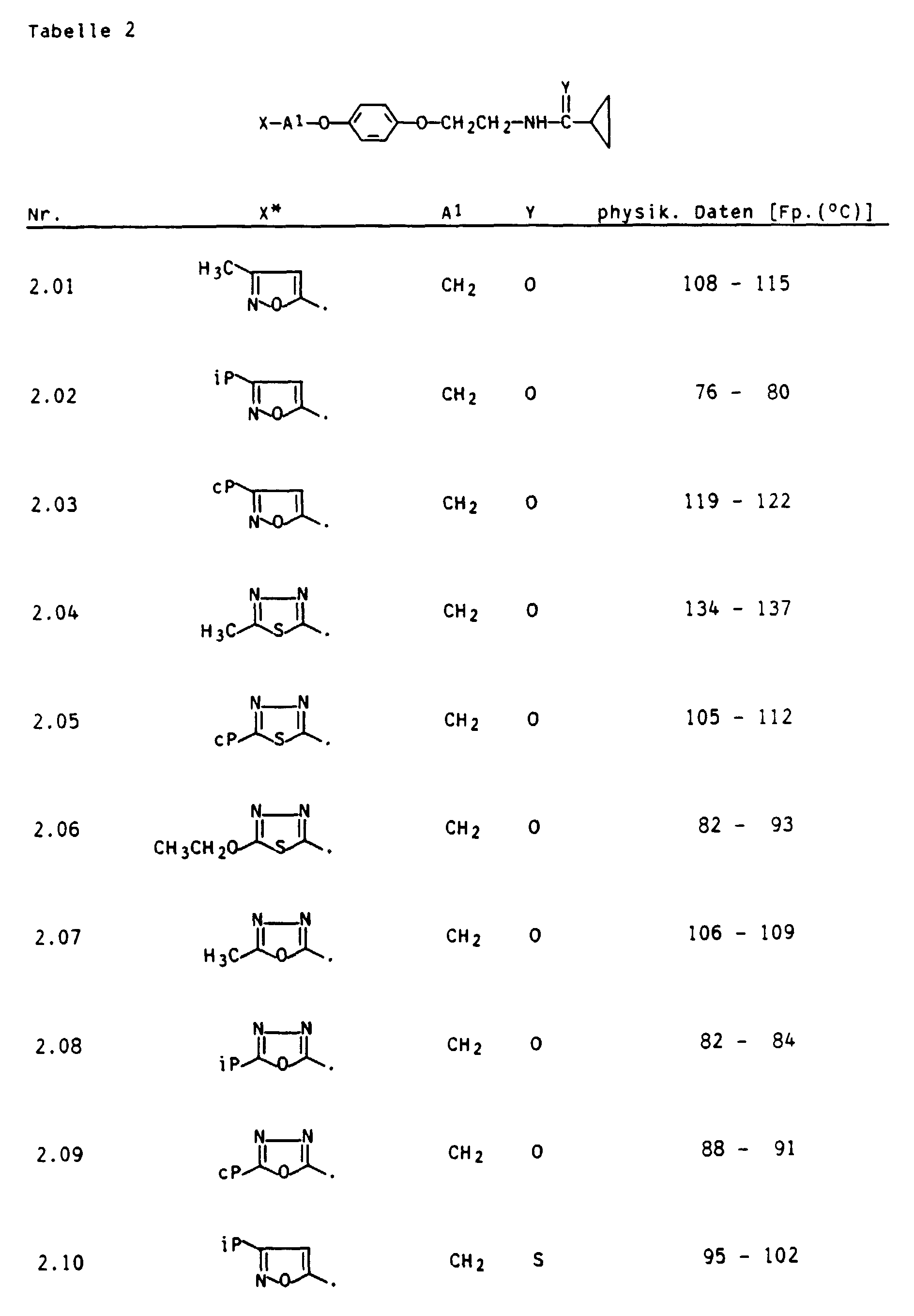

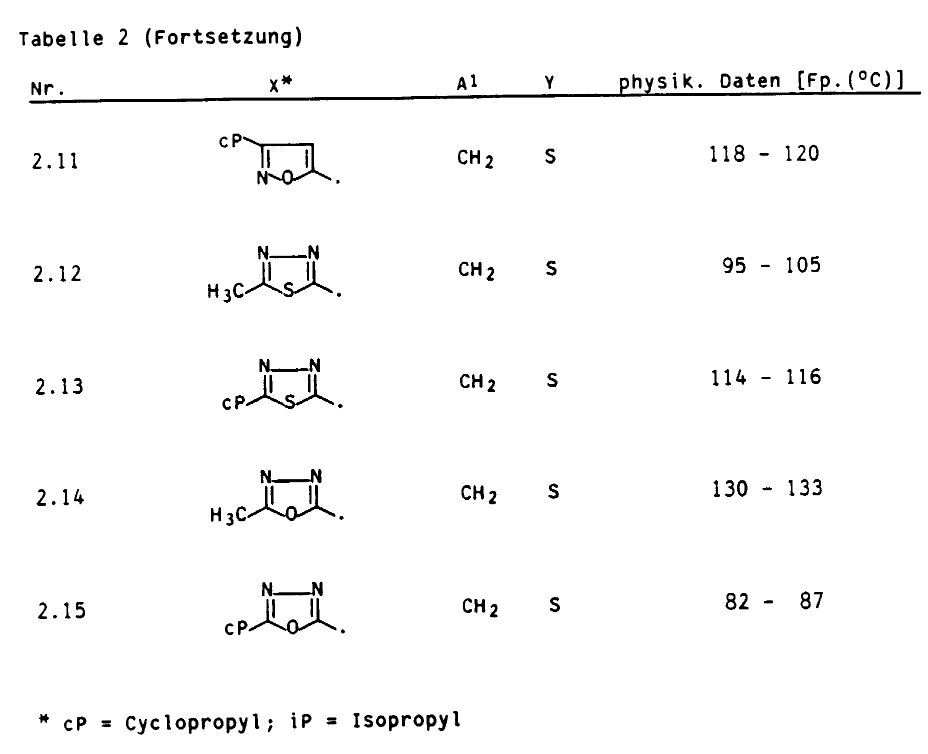

### Anwendungsbeispiele

Die insektizide Wirkung der Verbindungen der allgemeinen Formel I ließ sich durch folgende Versuche zeigen:

Die Wirkstoffe wurden
a) als 0,1 %-ige Lösung in Aceton oder
b) als 10 %-ige Emulsion in einem Gemisch aus 70 Gew.-% Cyclohexanol, 20 Gew.-% Nekanil® LN (Lutensol® AP6, Netzmittel mit Emulgier- und Dispergierwirkung auf der Basis ethoxylierter Alkylphenole) und 10 Gew.-% Emulphor® EL (Emulan® EL, Emulgator auf der Basis ethoxylierter Fettalkohole)
aufbereitet und entsprechend der gewünschten Konzentration mit Aceton im Fall von a) bzw. mit Wasser im Fall von b) verdünnt.

Nach Abschluß der Versuche wurde die jeweils niedrigste Konzentration ermittelt, bei der die Verbindungen im Vergleich zu unbehandelten Kontrollversuchen noch eine 80 - 100 %-ige Hemmung bzw. Mortalität hervorriefen (Wirkschwelle bzw. Minimalkonzentration).
A) Prodenia litura (Ägypt. Baumwollwurm), Zuchtversuch Der Boden eines Versuchsgefäßes wurde mit der acetonischen Lösung des Wirkstoffs benetzt und nach dem Abdampfen des Lösungsmittels mit 5 Raupen im 4. Larvenstadium besetzt. Nach 4 h wurde die Mortalität bestimmt. In diesem Test zeigten die Verbindungen 2.01, 2.04, 2.09, 2.13 und 2.15 Wirkschwellen von 0,0001 bis 0,0004 ppm.
B) Prodenia litura (Ägypt. Baumwollwurm), Morphogenese Fünf Raupen des Entwicklungsstadiums L3 (10-12 mm) wurden auf Standardnährboden (3,1 l Wasser, 80 g Agar, 137 g Bierhefe, 515 g Maismehl, 130 g Weizenkeime sowie übliche Zusatzstoffe und Vitamine (20 g Wessonsalz, 5 g Nipagin, 5 g Sorbin, 10 g Zellulose, 18 g Ascorbinsäure, 1 g Lutavit® blend (Vitamin), 5 ml alkoholische Biotin-Lösung) aufgebracht, der zuvor mit der wäßrigen Wirkstoffaufbereitung benetzt worden war.
   Die Beobachtung erstreckte sich bis zur Verpuppung in einem Kontrollversuch ohne Wirkstoff. Während der Dauer des Versuchs wurden auftretende Veränderungen in der Entwicklung im Vergleich zur Normalentwicklung beurteilt.
   In diesem Test zeigten die Verbindungen 2.01, 2.04, 2.09, 2.13 und 2.15 Wirkschwellen von 0,001 bis 0,0004 ppm.

## Patentansprüche

1. Cyclopropancarbonsäureamide und -thioamide der allgemeinen Formel I, 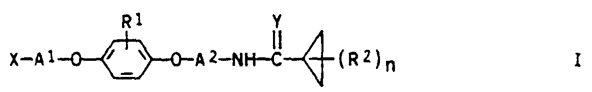 in der der Index und die Substituenten folgende Bedeutung haben:
n 0, 1 oder 2, wobei die Reste R² verschieden sein können, wenn n für 2 steht;
A¹ Methylen, Ethylen oder Propylen, wobei diese Gruppen ein oder zwei C₁-C₃-Alkylgruppen tragen können;
A² Ethylen oder Propylen, wobei diese Gruppen ein oder zwei C₁-C₃-Alkylgruppen tragen können;
R¹ Wasserstoff, Halogen oder C₁-C₆-Alkyl;
R² Halogen oder C₁-C₃-Alkyl;
X ein über ein C-Atom oder ein N-Atom gebundener 5-gliedriger heteroaromatischer Rest, enthaltend ein bis drei Stickstoffatome oder ein Sauerstoff- oder ein Schwefelatom oder enthaltend ein oder zwei Stickstoffatome und ein Sauerstoff- oder ein Schwefelatom als Heteroatome, oder ein Pyrazolyl-1-oxy- oder Triazolyl-1-oxyrest, wobei diese 5-Ring Heteroaromaten an ihren Kohlenstoffringgliedern ein bis drei der folgenden Reste tragen können: Nitro, Cyano, Halogen, C₁-C₆-Alkyl, C₁-C₄-Halogenalkyl, C₁-C₆-Alkoxy, C₁-C₄-Halogenalkoxy, C₁-C₄-Alkylthio, C₁-C₄-Halogenalkylthio, C₁-C₄-Alkoxy-C₁-C₄-alkyl, C₃-C₈-Cycloalkyl, C₂-C₈-Alkenyl, C₂-C₈-Alkinyl, Aryl oder Aryl-C₁-C₁₀-alkyl, wobei die aromatischen Reste ihrerseits ein bis fünf Halogenatome und/oder ein bis drei der folgenden Gruppen tragen können: C₁-C₄-Alkyl, C₁-C₄-Halogenalkyl, C₁-C₄-Alkoxy, C₁-C₄-Halogenalkoxy, C₁-C₄-Alkylthio oder C₁-C₄-Halogenalkylthio;
Y Sauerstoff oder Schwefel.

2. Verfahren zur Herstellung der Verbindungen der allgemeinen Formel I gemäß Anspruch 1, in denen Y Sauerstoff bedeutet, dadurch gekennzeichnet, daß man einen 4-Hydroxyphenolether der allgemeinen Formel II, 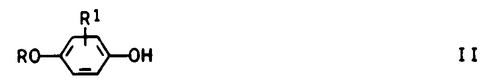 in der R für eine unter den Umsetzungsbedingungen inerte Schutzgruppe steht, in an sich bekannter Weise in einem inerten organischen Lösungsmittel in Gegenwart einer Base mit einem Nitril der allgemeinen Formel III,
Z¹-A³-CN III
in der Z¹ für eine nukleofuge Abgangsgruppe steht und A³ Methylen oder Ethylen bedeutet, welches ein oder zwei C₁-C₃-Alkylreste tragen kann, zu einem Hydrochinondiether der allgemeinen Formel IV 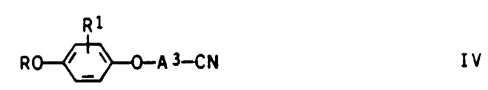 verethert, die Nitrilfunktion anschließend in an sich bekannter Weise reduziert, das so erhaltene primäre Amin der allgemeinen Formel V 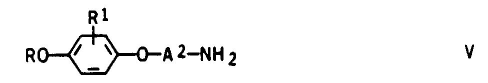 danach mit einer aktivierten Cyclopropancarbonsäure der allgemeinen Formel VI 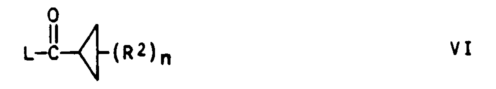 in der L für eine nukleophil verdrängbare Abgangsgruppe steht, in das entsprechende Amid der allgemeinen Formel VII 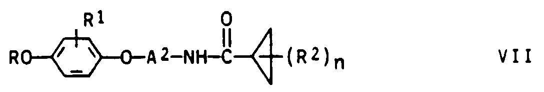 überführt, aus VII durch Abspaltung der Schutzgruppe R in an sich bekannter Weise das entsprechende Phenol der allgemeinen Formel VIII 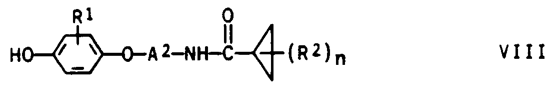 freisetzt, welches dann in an sich bekannter Weise mit einer Heteroarylalkylverbindung der allgemeinen Formel IX,
X-A¹-Z² IX
in der Z² für eine nucleofuge Abgangsgruppe steht, zu I verethert wird.

3. Verfahren zur Herstellung der Verbindungen der allgemeinen Formel I gemäß Anspruch 1, in denen Y Schwefel bedeutet, dadurch gekennzeichnet, daß man ein Cyclopropancarbonsäureamid der allgemeinen Formel I gemäß Anspruch 1, in der Y Sauerstoff bedeutet in an sich bekannter Weise in einem inerten organischen Lösungsmittel in Gegenwart eines Schwefel übertragenden Reagens umsetzt.

4. Verfahren zur Herstellung der Verbindungen der allgemeinen Formel I gemäß Anspruch 1, dadurch gekennzeichnet, daß man ein Amin der allgemeinen Formel X, 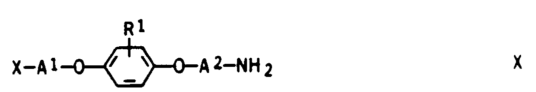 in an sich bekannter Weise in einem inerten organischen Lösungsmittel mit einer aktivierten Cyclopropancarbonsäure bzw. -thiocarbonsäure der allgemeinen Formel VI' 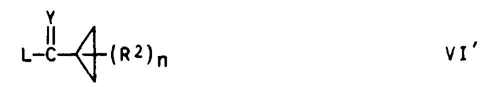 in der L für eine nukleophil verdrängbare Abgangsgruppe steht, umsetzt.

5. Phenole der allgemeinen Formel VIII gemäß Anspruch 2, in der der Index n und die Substituenten A², R¹ und R² die in Anspruch 1 gegebene Bedeutung haben.

6. Amine der allgemeinen Formel X gemäß Anspruch 4, in der die Substituenten A¹, A², R¹ und X die in Anspruch 1 gegebene Bedeutung haben.

7. Verfahren zu Herstellung der Amine der allgemeinen Formel X gemäß Anspruch 4, dadurch gekennzeichnet, daß man einen 4-Hydroxyphenolether der allgemeinen Formel II gemäß Anspruch 2 in an sich bekannter Weise mit einer Heteroarylalkylverbindung der allgemeinen Formel IX gemäß Anspruch 2 zu einem Diether der allgemeinen Formel V', 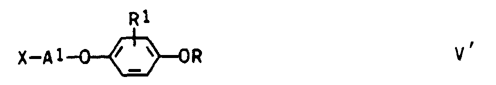 umsetzt, aus V' die Schutzgruppe abspaltet, das so erhaltene entsprechende Phenol der allgemeinen Formel VIII' 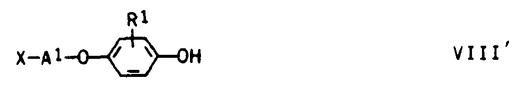 mit einem Nitril der allgemeinen Formel III gemäß Anspruch 2 zu einem Hydrochinondiether der allgemeinen Formel IV' 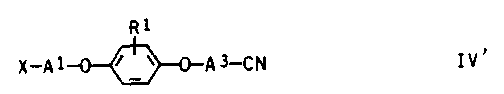 umsetzt und anschließend die Nitrilfunktion reduziert.

8. Schädlingsbekämpfungsmittel, enthaltend eine wirksame Menge einer Verbindung der allgemeinen Formel I gemäß Anspruch 1 sowie inerte Zusatzstoffe.

9. Verfahren zur Bekämpfung von Schädlingen, dadurch gekennzeichnet, daß man die Schädlinge und/oder ihren Lebensraum mit einer wirksamen Menge einer Verbindung der allgemeinen Formel I gemäß Anspruch 1 behandelt.

## Claims

1. A cyclopropane(thio)carboxamide of the formula I 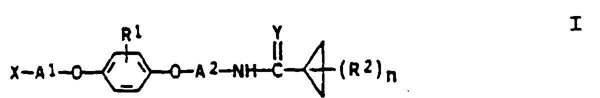 where
n is 0, 1 or 2, it being possible for the R² radicals to be different when n is 2;
A¹ is methylene, ethylene or propylene, it being possible for these groups to carry one or two C₁-C₃-alkyl groups;
A² is ethylene or propylene, it being possible for these groups to carry one or two C₁-C₃-alkyl- groups; R¹ is hydrogen, halogen or C₁-C₆-alkyl;
R² is halogen or C₁-C₃-alkyl;
X is a 5-membered heteroaromatic radical which is bonded via carbon or nitrogen and contains from one to three nitrogens or one oxygen or one sulfur or contains one or two nitrogens and one oxygen or one sulfur as hetero atoms, or is 1-pyrazolyloxy or 1-triazolyloxy, it being possible for these 5-membered heteroaromatic rings to carry on their carbon atoms from one to three of the following: nitro, cyano, halogen, C₁-C₆-alkyl, C₁-C₄-haloalkyl, C₁-C₆-alkoxy, C₁-C₄-haloalkoxy, C₁-C₄-alkylthio, C₁-C₄-haloahkylthio, C₁-C₄-alkoxy-C₁-C₄-alkyl, C₃-C₈-cycloalkyl, C₂-C₈-alkenyl, C₂-C₈-alkynyl, aryl or aryl-C₁-C₁₀-alkyl, it being possible for the aromatic radicals in turn to carry from one to five halogens and/or from one to three of the following: C₁-C₄-alkyl, C₁-C₄-haloalkyl, C₁-C₄-alkoxy C₁-C₄-haloalkoxy, C₁-C₄-alkylthio or C₁-C₄-haloalkylthio; C₁-C₄-alkylthio or C₁-C₄-haloalkylthio;
Y is oxygen or sulfur.

2. A process for preparing a compound of the formula I as claimed in claim 1, where Y is oxygen, which comprises etherifying a 4-hydroxyphenol ether of the formula II 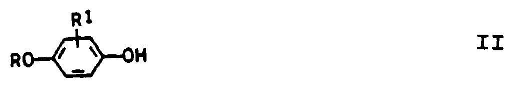 where R is a protective group which is inert under the reaction conditions, in a conventional manner in an inert organic solvent in the presence of a base with a nitrile of the formula III
Z¹-A³-CN III
where Z¹ is a nucleofugic leaving group, and A³ is methylene or ethylene which can carry one or two C₁-C₃-alkyl radicals, to give a hydroquinone diether of the formula IV 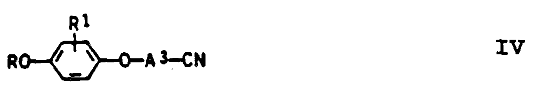 subsequently reducing the nitrile functionality in a conventional manner, then converting the resulting primary amine of the formula V 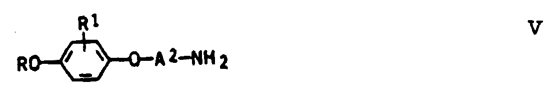 with an activated cyclopropanecarboxylic acid of the formula VI 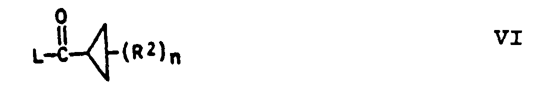 where L is a nucleophilically displaceable leaving group, into the corresponding amide of the formula VII 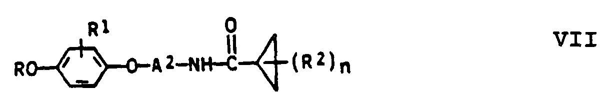 liberating from VII, by eliminating the protective group R in a conventional manner, the corresponding phenol of the formula VIII 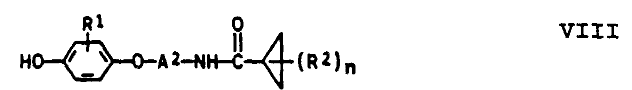 which is then etherified in a conventional manner with a heteroarylalkyl compound of the formula IX
X-A¹-Z² IX
where Z² is a nucleofugic leaving group, to give I.

3. A process for preparing a compound of the formula I as claimed in claim 1, where Y is sulfur, which comprises reacting a cyclopropanecarboxamide of the formula I as claimed in claim 1, where Y is oxygen, in a conventional manner in an inert organic solvent in the presence of a sulfur-transferring reagent.

4. A process for preparing a compound of the formula I as claimed in claim 1, which comprises reacting an amine of the formula X 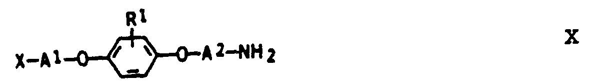 in a conventional manner in an inert organic solvent with an activated cyclopropane(thio)carboxylic acid of the formula VI' 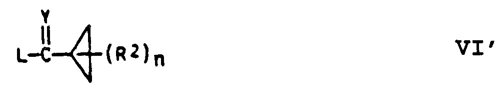 where L is a nucleophilically displaceable leaving group.

5. A phenol of the formula VIII as claimed in claim 2, where n and A², R¹ and R² have the meanings given in claim 1.

6. An amine of the formula X as claimed in claim 4, where A¹, A², R¹ and X have the meanings given in claim 1.

7. A process for preparing an amine of the formula X as claimed in claim 4, which comprises reacting a 4-hydroxyphenol ether of the formula II as claimed in claim 2 in a conventional manner with a heteroarylalkyl compound of the formula IX as claimed in claim 2 to give a diether of the formula V' 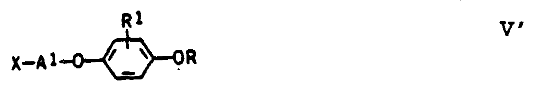 eliminating the protective group from V', reacting the resulting phenol of the formula VIII' 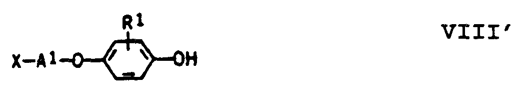 with a nitrile of the formula III as claimed in claim 2 to give a hydroquinone diether of the formula IV' 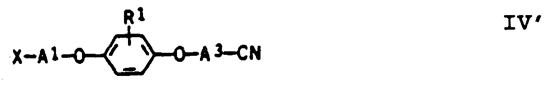 and subsequently reducing the nitrile functionality.

8. A pesticide containing an effective amount of a compound of the formula I as claimed in claim 1 and inert additives.

9. A method for controlling pests, which comprises treating the pests and/or their habitat with an effective amount of a compound of the formula I as claimed in claim 1.

## Revendications

1. Amides et thioamides d'acide cyclopropanecarboxylique de formule générale I, 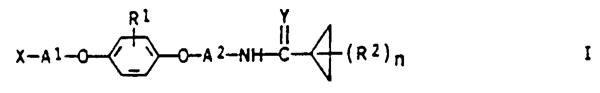 où l'indice et les substituants ont la signification suivante:
n vaut 0, 1 ou 2, tandis que les restes R² peuvent être différents quand n vaut 2;
A¹ méthylène, éthylène ou propylène, tandis que ces groupes peuvent porter un ou deux groupes alkyle en C₁-C₃;
A² éthylène ou propylène, tandis que ces groupes peuvent porter un ou deux groupes alkyle en C₁-C₃;
R¹ hydrogène, halogène ou alkyle en C₁-C₆;
R² halogène ou alkyle en C₁-C₃;
X un reste hétéroaromatique à 5 chaînons lié par l'intermédiaire d'un atome C ou d'un atome N, contenant un à trois atomes d'azote ou un atome d'oxygène ou un atome de soufre ou contenant un ou deux atomes d'azote et un atome d'oxygène ou un atome de soufre comme hétéroatomes, ou un reste pyrazolyl-1-oxy ou triazolyl-1-oxy, tandis que ces composés hétéroatomatiques à 5 chaînons peuvent porter sur leurs chaînons cycliques hydrocarbonés un à trois des restes suivants: nitro, cyano, halogène, alkyle en C₁-C₆, halogénoalkyle en C₁-C₄, alcoxy en C₁-C₆, halogénoalcoxy en C₁-C₄, alkylthio en C₁-C₄, halogénoalkylthio en C₁-C₄, alcoxy(C₁-C₄)alkyle(C₁-C₄), cycloalkyle en C₃-C₈, alcényle en C₂-C₈, alcynyle en C₂-C₈, aryle ou aryl-alkyle(C₁-C₁₀), tandis que les restes aromatiques peuvent porter à leur tour un à cinq atomes d'halogène et/ou un à trois des groupes suivants: alkyle en C₁-C₄, halogénoalkyle en C₁-C₄, alcoxy en C₁-C₄, halogénoalcoxy en C₁-C₄, alkylthio en C₁-C₄ ou halogénoalkylthio en C₁-C₄;
Y oxygène ou soufre.

2. Procédé pour la préparation des composés de formule générale I selon la revendication 1, dans lesquels Y représente l'oxygène, caractérisé par le fait qu'on éthérifie un éther de 4-hydroxyphénol de formule générale II, 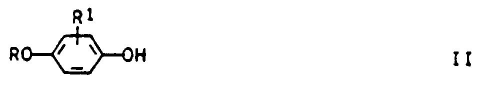 où R désigne un groupe protecteur inerte dans les conditions de la réaction, de manière connue en soi dans un solvant organique inerte, en présence d'une base, avec un nitrile de formule générale III,
Z¹-A³-CN III
où Z¹ représente un groupe séparable nucléofuge et A³ désigne le méthylène ou l'éthylène, qui peut porter un ou deux restes alkyle en C₁-C₃, pour former un diéther d'hydroquinone de formule générale IV 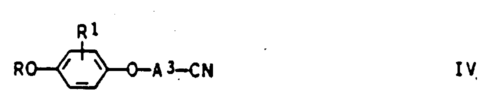 on réduit ensuite la fonction nitrile de manière connue en soi, on convertit après cela l'amine primaire ainsi obtenue de formule générale V 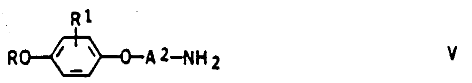 avec un acide cyclopropanecarboxylique activé de formule générale VI 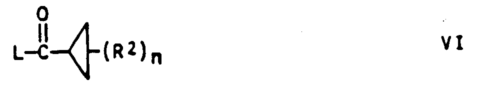 où L désigne un groupe séparable déplaçable par voie nucléophile, en l'amide correspondant de formule générale VII 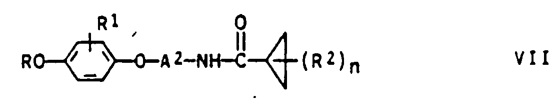 on libère de VII, par élimination du groupe protecteur R, de manière connue en soi, le phénol correspondant de formule générale VIII 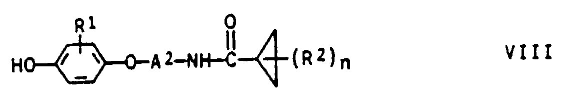 qui est ensuite éthérifié en I, d'une manière connue en soi, avec un composé hétéroarylalkylique de formule générale IX,
X-A¹-Z² IX
où z² désigne un groupe éliminable nucléofuge.

3. Procédé pour la préparation de composés de formule générale I selon la revendication 1, dans lesquels Y représente le soufre, caractérisé par le fait qu'on fait réagir un amide d'acide cyclopropanecarboxylique de formule générale I selon la revendication 1, où Y désigne l'oxygène, d'une manière connue en soi, dans un solvant organique inerte, en présence d'un réactif transférant du soufre.

4. Procédé pour la préparation des composés de formule générale I selon la revendication 1, caractérisé par le fait qu'on fait réagir une amine de formule générale X 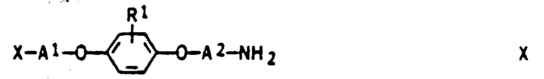 de manière connue en soi, dans un solvant organique inerte, avec un acide cyclopropanecarboxylique ou -thiocarboxylique de formule générale VI' 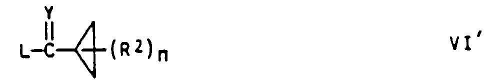 où L désigne un groupe éliminable séparable par voie nucléophile.

5. Phénols de formule générale VIII selon la revendication 2, dans lesquels l'indice n et les substituants A², R¹ et R² ont la signification indiquée dans la revendication 1.

6. Amines de formule générale X selon la revendication 4, dans lesquelles les substituants A¹, A², R¹ et X ont la signification indiquée dans la revendication 1.

7. Procédé pour la préparation des amines de formule générale X selon la revendication 4, caractérisé par le fait qu'on fait réagir un éther de 4-hydroxyphénol de formule générale II selon la revendication 2, d'une manière connue en soi, avec un composé hétéroarylalkylique de formule générale IX selon la revendication 2 pour former un diéther de formule générale V', 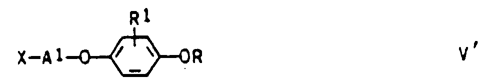 on élimine le groupe protecteur de V', on fait réagir le phénol correspondant ainsi obtenu de formule générale VIII' 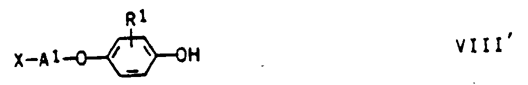 avec un nitrile de formule générale III selon la revendication 2, pour former un diéther d'hydroquinone de formule générale IV' 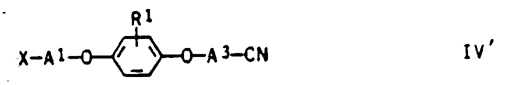 et on réduit ensuite la fonction nitrile.

8. Agent de lutte contre les parasites, contenant une quantité efficace d'un composé de formule générale I selon la revendication 1, ainsi que des additifs inertes.

9. Procédé pour la lutte contre les parasites, caractérisé par le fait qu'on traite les parasites et/ou leur biotope avec une quantité efficace d'un composé de formule générale I selon la revendication 1.
